(19) Europäisches Patentamt
European Patent Office
Office européen des brevets

(11) EP 4 134 672 A1

(12) # EUROPEAN PATENT APPLICATION

(43) Date of publication:
**15.02.2023 Bulletin 2023/07**

(21) Application number: **21190496.6**

(22) Date of filing: **10.08.2021**

(51) International Patent Classification (IPC):
***G01N 33/68*** *(1980.01)*

(52) Cooperative Patent Classification (CPC):
**G01N 33/6893; G01N 33/6848;** G01N 2333/90638; G01N 2800/04

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Immundiagnostik AG**
**64625 Bensheim (DE)**

(72) Inventors:
- **DIESNER, Max**
**64625 Bensheim (DE)**
- **ARMBRUSTER, Franz Paul**
**64270 Bobenheim-Roxheim (DE)**

(74) Representative: **Benedum, Ulrich Max**
**Nebens IP**
**Patente Marken Designs**
**Oberföhringer Strasse 172**
**81925 München (DE)**

(54) **DIFFERENTIAL DIAGNOSIS OF HISTAMINE INTOLERANCE SYNDROME**

(57) A method for diagnosing histamine intolerance syndrome in a patient suspected of suffering from insufficient histamine inactivation activity. The diagnostic procedure involves spiking or administering of histamine labelled with a stable isotope. Samples (usually serum, plasma, urine) are taken after one or more predetermined periods of time and an aprotic solvent is added to remove proteins and organic salts. The amounts of isotopically labelled histamine in the samples and, if applicable, one or more isotopically labelled inactivation products of histamine are determined by hydrophilic interaction chromatography and mass spectrometry. The histamine inactivation activity can then be determined from the amounts and ratios of isotopically labelled histamine, imidazole acetic acid, and methylhistamine, and optionally methylimidazole acetic acid. Diagnosis is based on the comparison with the histamine inactivation found in healthy individuals.

**Fig. 1**

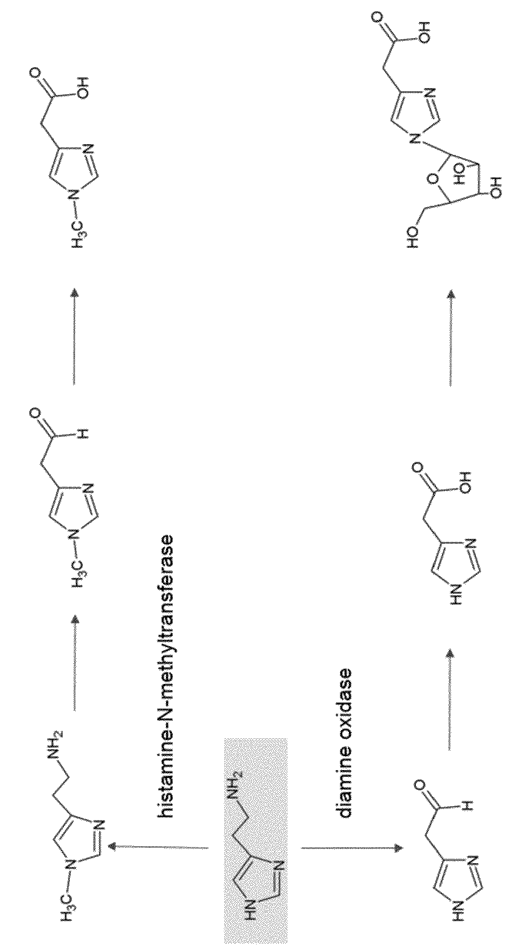


EP 4 134 672 A1

## Description

### FIELD OF THE INVENTION

The present invention relates to a method and reagents for a differential diagnosis of histamine intolerance syndrome (WHO ICD10 - T78.1).

### BACKGROUND OF THE INVENTION

Histamine intolerance, also referred to as enteral histaminosis or sensitivity to dietary histamine, is a disorder associated with an impaired ability to metabolize histamine. Histamine [2-(1H-imidazol-4-yl)-ethan-1-amine] is formed in the body from L-histidine which is decarboxylated by the action of histidine decarboxylase (HDC, EC 4.1.1.22). This is the only endogenous source of histamine. The main functions of histamine are mediated through four different receptors (H1R-H4R) in humans (Cataldi et al., Histamine receptors and antihistamines: from discovery to clinical applications. 2014, Chem Immunol Allergy. 2014; 100:214-26). Histamine in general takes part in physiological and pathophysiological processes connected with neurotransmission, gastric acid secretion, immune responses, and Inflammatory reactions of any kind. As histamine is physiologically highly active, its levels are physiologically tightly controlled at all stages: at intercellular transport, storage in secretory granules, release and by its inactivation and inactivation.

Histamine is degraded in mammals by two pathways as shown in Fig.1:-

(1) enzymatic degradation to imidazole-4-acetaldehyde [2-(1H-imidazol-5-yl)acetaldehyde] by secreted copper-containing (AOC1) diamine oxidase (DAO; EC 1.4.3.22), which is followed by a second enzymatic step mediated by mammalian aldehyde dehydrogenases (ADH; EC 1.2.1.3) that convert imidazole-4-acetaldehyde into imidazole acetic acid [2-(1H-imidazol-5-yl)acetic acid];

(2) enzymatic degradation by histamine N-methyltransferase (HNMT; EC 2.1.1.8) into 1-methylhistamine [2-(1-methyl-1H-imidazol-4-yl)ethan-1-amine], which is followed by a second enzymatic step that converts 1-methylhistamine into methylimidazole acetaldehyde [2-(1-methyl-1H-imidazol-4-yl)acetaldehyde] and mediated by monoamine oxidase A (MAO-A; EC 1.4.3.4) and by a third enzymatic step mediated by aldehyde dehydrogenase (ALDHIII; EC 1.2.1.5) resulting in methylimidazole acetic acid [2-(1-methyl-1H-imidazol-4-yl)acetic acid] as final product.

Histamine intolerance syndrome is difficult to diagnose because of its multiple symptoms which are easily misinterpreted as symptoms of other diseases, e.g., food allergy, sulphide intolerance, or as caused by other biogenic amines such as tyramine. Histamine intolerance causes painful symptoms and is a common condition affecting about 1%-3% of the population (Sattler J et al., Food induced histaminosis as an epidemiological problem: plasma histamine elevation and hemodynamic alterations after oral histamine administration and blockade of diamine oxidase (DAO), Agents and Actions 1988, 23:361-65; Wantke F et al., The red wine maximization test: drinking histamine rich wine induces a transient increase of plasma diamine oxidase activity in healthy volunteers, Inflamm Res 1999, 48:169-70; Wantke F et al., The red wine provocation test: intolerance to histamine as a model for food intolerance, Allergy Proc 1994, 15:27-32; Wantke F et al., Daily variations of serum diamine oxidase and the influence of H1 and H2 blockers: a critical approach to routine diamine oxidase assessment, Inflamm Res 1998, 47:396-400; Jarisch R et al., Role of food allergy and food intolerance in recurrent urticaria, Curr Probl Dermatol 1999, 28:64-73, Wantke F et al., Histamine free diet: treatment of choice for histamine induced food intolerance and supporting treatment for chronical headaches, Clin Exp Allergy 1993, 23:982-85; Götz M et al., Histamin-Intoleranz und Diaminooxidasemangel, Allergologie 1996, 9:426-30; Jarisch R et al., Histamin-Intoleranz. Histamin und Seekrankheit. Stuttgart, NY, 2 ed., Georg Thieme Verl., 2004).

The secreted copper-containing (AOC1) diamine oxidase (DAO- EC 1.4.3.22) is generally regarded the frontline enzyme in the clearing of endogenous and exogenous histamine since blood leukocytes and tissues such as the placenta secret DAO into the circulation. Pregnant women for example have serum DAO levels 500 to 1000 times higher than non-pregnant women. Humans have three genes encoding copper-containing amine oxidases which share large sequence identity but only one gene, the AOC1 gene, encodes secreted human diamine oxidase (McGrath AP et al, Structure and Inhibition of Human Diamine Oxidase, Biochemistry 2009; 48:9810-22). Dietary histamine is primarily degraded by tissue-associated DAO in the intestinal mucous membrane when it passes therethrough. Tissue-associated DAO is not merely found in the small intestine but also in liver, lung, and kidneys, and amniotic fluid contains a diamine oxidase which is immunologically different to the diamine oxidase (DAO) found in serum; see Cowley et al in US 5,284,749 A; Elmore BO et al., Human kidney diamine oxidase: heterologous expression, purification, and characterization, J Biol Inorg Chem 2002, 7:565- 679; Mizuguchi et al., Purification and characterization of diamine oxidase (histaminase) from rat small intestine, J. Biochem 1994,116:631-5; Kitanaka et al., Expression of diamine oxidase (histaminase) in guinea-pig tissues, Eur J Pharmacol 2002, 437:179-85). Human urine contains a higher or at least a similarly high concentration of methylimidazole acetic acid compared with imidazole acetic acid. It has therefore been suspected and shown that

the HNMT inactivation pathway [degradation pathway (2)], in addition to the secreted and tissue-bound DAO degradation pathways plays a crucial role in histamine degradation and may be significantly involved in the case of an impaired histamine degradation capacity (Khandelwal et al., Presence and measurement of methylimidazole acetic acids in brain and body fluids, J Biol Chem. 1982 Nov 10;257(21):12815-9; Tsuruta et al., Simultaneous determination of imidazole acetic acid and N tau- and N pi-methylimidazole acetic acids in human urine by high-performance liquid chromatography with fluorescence detection, J Chromatogr. 1987 Apr 24;416(1):63-9; Preuss et al., Human histamine N-methyl-transferase pharmaco-genetics: common genetic polymorphisms that alter activity, Molecular Pharmacology April 1, 1998 vol. 53 no. 4 708-717; Rutherford et al., The histamine N-methyltransferase T105I polymorphism affects active site structure and dynamics, Biochemistry. 2008 Jan 22;47(3):893-901; Lee HS et al., Involvement of human histamine N-methyl-transferase gene poly-morphisms in susceptibility to atopic dermatitis in korean children, Allergy Asthma Immunol Res. 2012 Jan;4(1):31-6; Nelis et al., Development of a HILIC-MS/MS method for the quantification of histamine and its main metabolites in human urine samples, Talanta Vol. 220, 1 December 2020, 121328). HNMT is localized in the cell cytosol, present in tissues throughout the body, and expressed in macrophages and monocytes as well (www.proteinatlas.org/ENSG00000150540-HNMT/tissue). The histamine degradation pathways therefore compete and overlap, making a differential diagnosis difficult of which degradation pathway is impaired.

Conventional diagnostic methods use a diamine/histamine conversion ratio and a diamine/histamine conversion rate per unit time based on a measurement of histamine in plasma or serum. US 4,818,683 (Morel et al) describes an immunoassay which measures a histamine derivative. Alternative methods of measuring histamine have been described by Ellman GL, Arch Biochem Biophys 1958, 74:443-450; Riddles PW, Anal Biochem 1979, 94:75-81; Muckerheide A et al., J. Immunol 1987, 138:833; Apple RJ et al, J Immunol 1987, 140:290; Domen PJ, J Immunol 1987,139:195; Morel and Delaage, Immuno analysis of histamine through a novel chemical derivatization, The Journal of Allergy and Clinical Immunology 1988, 82:646-654; Morel et al., Recognition of imidazole and histamine derivatives by monoclonal antibodies, Immunol. 1990 27:995-1000; Held, P. et al. Analysis of Histamine in Wine Samples Using the Microplate Format. BioTek Instruments Aug. 28, 2006).

EP 1 948 819 (Sciotec Diagnostic Technologies GmbH) relates to a method for determining a DAO activity in plasma or serum. An excess of a diamine substrate, e.g., putrescine, is added to the sample and reacted by the intrinsic DAO in said sample for a defined period. The diamine being still in the sample is derivatized and determined by a competitive immunoassay. As the diamine substrate concentration stays at elevated level, the rate of DAO is proportional to its concentration in the sample and used for diagnosis. Immunoassays (ELISA) for directly measuring the amount of DAO enzyme in human serum and dried blood spots as well as of the DAO activity ($^3$H putrescine/pyrroline REA) are available from Immundiagnostik AG, Bensheim, DE (IDK® DAO ELISA - Art. No. K8500; Art. No. K 8220); Mayer I et al., Optimierter Radioextraktionsassay zur quantitativen Bestimmung der Aktivität von Diaminooxidase (DAO) in humanem Serum und Plasma, Allergologie 2005, 28:1-8).

WO 2019/158718 (Frost Diagnostika GmbH) suggests determining an index ("percentage of the total histamine degradation") to combine the decreasing DAO rate and the decreasing diamine substrate level in a method as described in EP 1948819 B1. This index is said to be more useful for diagnosis of a histamine intolerance syndrome.

EP1948819 B1 and Appl Biochem Biotechnol 2007 Nov;143(2):164-75 describe a method wherein the DAO in a sample is captured by an immobilized antibody. A diamine substrate is added to the immobilized DAO and then reacted which results in a rise of hydrogen peroxide (Hibi T et al, Enzymatic assay of histamine by amperometric detection of H2O2 with a peroxidase-based sensor, Biosci Biotechnol Biochem 2000, 64(9):1963-1966).

Further background with respect to the above can be found in: Kehoe CA et al., Plasma diamine oxidase activity is greater in copper-adequate than copper-marginal or copper-deficient rats, J. Nutr 2000, 130:30-33; Küfner MA et al, Total histamine degradation capacity (THDC) as an important biological marker of histamine metabolism in human colonic mucosa, Inflamm Res 2002, Suppl 1:87-81; Nilsson B-O et al., Inhibition of diamine oxidase promotes uptake of putrescine from rat small intestine, Inflamm Res 1996, 45:513-518; Novotny et al., Diamine oxidase is the amiloride-binding protein and is inhibited by biological marker of histamine metabolism in human colonic mucosa, Inflamm Res 2002, Suppl 1:87-81; Nilsson B-O et al., Inhibition of diamine oxidase promotes uptake of putrescine from rat small intestine, Inflamm Res 1996, 45:513-518; Novotny et al., Diamine oxidase is the amiloride-binding protein and is inhibited by products, Eur J Biochem, 2004, 271:146-152.

The current methods for determining the diamine/histamine conversion ratio and a diamine/histamine conversion rate per unit time and for diagnosis of histamine intolerance syndrome are all based on a measurement of serum DAO or DAO activity in blood, plasma, or serum or a measurement of one or more catabolites of histamine. A differential diagnosis of the origin or cause of the histamine intolerance syndrome is not possible. The state of the art represents a problem.

## BRIEF DESCRIPTION OF THE INVENTION

The problem is partly overcome by a method of determining the histamine inactivation in a human subject

suspected of suffering from histamine intolerance syndrome and/or insufficient histamine inactivation activity. The method of diagnosis according to the invention comprises the steps of:--

administering a preparation, solution, or suspension containing a known amount of histamine, labelled by a stable isotope;
obtaining samples of bodily fluids selected from blood, serum, plasma, urine, or saliva after one or more predetermined periods of time;
adding an aprotic solvent miscible with the aqueous sample to the samples and removing proteins and organic salts from the samples;
determining the amounts of isotopically labelled histamine, methyl histamine and imidazole acetic acid in said sample using hydrophilic interaction chromatography and mass spectrometry which comprises an LC-MS/MS technique; and
calculating the histamine inactivation activity of the subject on basis of the amounts of isotopically labelled histamine, imidazole acetic acid and methyl histamine; and comparing the found histamine inactivation activity with the histamine inactivation activity found in a healthy subject.

In some embodiments, the method further comprises a determination of isotopically labelled methyl imidazole acetic acid in the sample, and optionally, an immunological determination of secreted human diamine oxidase in serum or plasma.

According to the invention, the isotopically labelled histamine is selected from histamine $^{15}N$-labelled at positions at N1 and/or N3 or a histamine $^{13}C$-labelled at positions C2, C4, C5, C6, or C7 or a histamine deuterium labelled at anyone or more hydrogen position, or a histamine containing a combination of stable isotope labels.

The aprotic solvent may be selected from acetonitrile, tetrahydrofuran, acrylonitrile, dioxane, ethanol but preferably acetonitrile is preferred solvent because it works equally with zwitterionic HILIC LC columns, PEI HILIC LC columns, silica HILIC columns and urea HILIC columns, as well as with amide HILIC columns as well as with reversed phase columns. It is commonly believed that in HILIC, the mobile phase forms a water-rich layer on the surface of the polar stationary phase vs. the water-deficient mobile phase, creating a liquid/liquid extraction system. The analyte is distributed between these two layers. However, HILIC is more than a simple partitioning and includes hydrogen donor interactions between neutral polar species as well as weak electrostatic mechanisms under the high organic solvent conditions used for retention. This distinguishes HILIC as a mechanism distinct from ion exchange chromatography. The more polar compounds have a stronger interaction with the stationary aqueous layer than the less polar compounds. Thus, a separation based on a compound's polarity and degree of solvation takes place for which acetonitrile is an ideal mobile phase.

In some embodiments, the method comprises a determination of any one of the isotope labelled ratios selected from relative amounts of

histamine / methylhistamine,
histamine / imidazole acetic acid,
methyl histamine / methyl imidazole acetic acid;
histamine /

methyl histamine + imidazole acetic acid + methyl imidazole acetic acid

and
optionally, further a diagnosis of which route of histamine inactivation is inhibited or deficient. This will allow a more efficient treatment of the histamine intolerance syndrome.

Another aspect of the invention relates to a method of diagnosis of histamine intolerance syndrome in a subject suspected of suffering from insufficient secreted DAO activity, and facultatively, of insufficient histamine N-methyl transferase (HNMT) activity, comprising the steps of:-

obtaining a sample of blood, plasma or serum from said subject suspected of suffering from histamine intolerance;
adding to said sample a predefined amount of stable isotopically labelled histamine to produce a defined concentration of isotopically labelled histamine in said sample, wherein the isotopically labelled histamine is selected from histamine $^{15}N$-labelled at positions at N1 and/or N3 or a histamine $^{13}C$-labelled at positions C2, C4, C5, C6, or C7 or a histamine deuterium labelled at anyone or more hydrogen position, or a histamine containing a combination of stable isotope labels;
incubation of said sample for a predefined period under physiological conditions to obtain inactivation of said isotopically labelled histamine by the activity of DAO and HNMT enzyme contained in said sample of blood, plasma or serum;

adding an aprotic solvent, miscible with the aqueous sample, to the samples, and removing proteins and organic salts from the samples;

determining the amounts of isotopically labelled histamine in said sample and one or more other isotopically labelled inactivation products of histamine using hydrophilic interaction chromatography and mass spectrometry which comprises an LC-MS/MS technique; and

calculating the histamine inactivation activity and/or the DAO activity and/or the HNMT activity in blood, plasma or serum of the subject on basis of the amounts of isotope-labelled histamine and its respective catabolites imidazole acetic acid, or methyl histamine, and methyl imidazole acetic acid.

This method typically comprises a spiking of the sample with an isotope-labelled histamine to achieve a spiked-histamine concentration of from 200 to 500 nmol·litre-1 serum.

In some embodiments, the method may comprise a calculating of the activity of soluble human DAO enzyme in said sample at given physiological conditions from the amounts of isotopically labelled histamine substrate converted per unit of time into imidazole acetic acid and determining the histamine inactivation capacity or half-life of histamine in said patient, to diagnose a histamine intolerance when the patient's diamine oxidase activity is below 3 enzyme units (U) per milliliter serum or plasma or below 10 enzyme units (U) per milliliter as measured by the immunoassay and/or the half-life of carbon-13 histamine in serum is above a threshold found in a sample of a healthy subject.

In some embodiments, the method may further comprise an immunological determination of soluble human diamine oxidase in serum or plasma and a determination of the enzyme activity of DAO in serum or plasma.

A further aspect of the invention relates to a kit for diagnosis of histamine intolerance syndrome, comprising defined histamine oral load in the form of a solution, dispersion or tablet, which contains a defined amount of stable isotope labelled histamine selected from histamine $^{15}N$-labelled at positions N1 and/or N3 or a histamine $^{13}C$-labelled at positions C2, C4, C5, C6, or C7 or a histamine deuterium labelled at one or more hydrogen positions; and solutions containing a stable isotope labelled standard, selected from proposed isotopically labelled histamine, optionally from imidazole acetic acid, methyl histamine and methyl imidazole acetic (see formula I-IV).

Another aspect of the invention relates to a kit for diagnosis of histamine intolerance syndrome in a sample of plasma or serum comprising a histamine spiking solution, which contains a defined amount of stable isotope labelled histamine selected from histamine $^{15}N$-labelled at positions N1 and/or N3 or a histamine $^{13}C$-labelled at positions C2, C4, C5, C6, or C7 or a histamine deuterium labelled at anyone or more hydrogen position, or a histamine having a combination of stable isotope labels, and solutions containing stable isotope labelled standards of the respective catabolites, selected from proposed isotopically labelled positions of histamine, imidazole acetic acid, methyl histamine and methyl imidazole acetic (see formula I-IV). In some embodiments, the kit may comprise an enzyme solution of catalase, peroxidase, and/or alcohol dehydrogenase.

The advantages of the above methods and the method of diagnosis are on the one hand its simplicity and diagnostically, that it reflects the physiological situation and simultaneously the combined routes of histamine inactivation either via methyl histamine and/or methylimidazole acetic acid or via imidazole acetic acid. Consequently, it also detects any interference by a lack of or inhibition of the aldehyde dehydrogenase and the alcohol detoxification mechanism.

The invention will be further described with respect to its advantages, embodiments, specific examples, and drawings which shall not be considered limiting. Reference to any prior art in the detailed description is not an acknowledgement or suggestion that this prior art forms part of the common general knowledge or that this prior art could reasonably be expected to be combined with any other piece of prior art by a skilled person in the art. By way of clarification and for avoidance of doubt, as used herein and except where the context requires otherwise, the term "comprise" and variations of the term, such as "comprising", "comprises" and "comprised", are not intended to exclude further additions, components, integers, or steps. The scope of the invention is described in the claims.

BRIEF DESCRIPTION OF DRAWING

It is shown in: -

Fig. 1 a schematic representation of the two histamine degradation pathways;

**Fig. 2** diagram of a HILIC gradient used for LC-MS/MS analysis of the degradation pathways of histamine and its catabolites;

**Fig. 3** a representative chromatogram of histamine, methyl histamine and corresponding imidazole catabolites from a spiked and analyzed serum sample (c = 10ng/ml per analyte).

**Fig. 4** a representative full scan spectrum of fragmented unlabelled histamine *(m/z* 112,117 $[M+H]^+$);

**Fig. 5** a representative full scan spectrum of fragmented *d4* labelled histamine (*m/z* 116,133 $[M+H]^+$);

**Fig. 6** a representative full scan spectrum of fragmented $^{13}C_5$ histamine *(m/z* 117,113 $[M+H]^+$);

**Fig. 7** a representative full scan spectrum of fragmented unlabelled methylhistamine *(m/z* 126,128 $[M+H]^+$);

**Fig. 8** a representative full scan spectrum of fragmented $^{13}C_5$ labelled methyl histamine *(m/z* 131,128 $[M+H]^+$);

**Fig. 9** a representative full scan spectrum of fragmented unlabelled imidazole acetic acid *(m/z* 127,069 $[M+H]^+$);

**Fig. 10** a representative full scan spectrum of fragmented $^{13}C_5$ labelled imidazole acetic acid *(m/z* 132,073 $[M+H]^+$);

**Fig. 11** a representative full scan spectrum of fragmented unlabelled methylimidazole acetic acid *(m/z* 141,093 $[M+H]^+$);

**Fig. 12** a representative full scan spectrum of fragmented $^{13}C_5$ labelled methylimidazole acetic acid (m/z 146,088 $[M+H]^+$).

## DETAILED DESCRIPTION OF THE INVENTION

Histamine is present in many fermented foods and beverages and their consumption at elevated levels can trigger pseudoallergic reactions and leads to numerous adverse conditions, including asthmatic wheezing, swelling of eyelids, eye turgor and respiratory mucosa, flushing, skin reddening, rushes, urticaria, pruritus. Consumed histamine may also lead to a lowering of the blood pressure and a contraction of the smooth muscles in the intestine and respiratory tract, may trigger gastrointestinal problems, abdominal pain, constipation, diarrhoea, migraine, headache, arrhythmia, oedema, and sleep disturbances.

Histamine intolerance syndrome is observed in cases of increased consumption or an overproduction of endogenous histamine under certain conditions, e.g. allergies, mastocytosis, bacterial infections, gastrointestinal bleedings. The consumption of alcohol may also lead to a histamine intolerance syndrome since the alcohol and histamine metabolic pathways have the common enzymes aldehyde dehydrogenase and aldehyde oxygenase (Zimatkin SM et al, Alcohol-histamine interactions, Alcohol Alcohol 1999, 34(2):151-7).

The inactivation of histamine inactivation may be inhibited or compromised by a lack of co-factors such as vitamin C, vitamin B6, copper or manganese ions or the presence of competing biogenic diamines. It is further known that the inactivation of histamine can be inhibited by drugs, including muscle relaxants (pancuronium, alcuronium, D-tubocurarine), narcotics (thiopental), analgetics (morphine, pethidine, non-steroidal and anti-inflammatory drugs (acetylsalicylic acid, metamizole), local anaesthetics (prilocaine), antihypotonics (dobutamine), antihypertensive drugs (verapamil, alprenolol, dihydralazin), antiarrhythmics (propafenone), diuretics (amiloride), drugs influencing gut motility (metoclopramide), antibiotics (cefuroxime, cefotiam, isoniazid, pentamidin, clavulanic acid, chloroquine), mucolytics (acetylcysteine, ambroxol), broncholytics (aminophylline), H2-receptor antagonists (cimetidine), cytostatics (cyclophosphamide), antidepressants (amitriptyline). DAO levels in humans further increase sharply within 30 minutes of heparin administration. Thus, there is a need to determine at which level the histamine inactivation is impaired, which implies that all routes of histamine inactivation must be studied and examined.

As mentioned in the background section, there are two equivalent pathways for histamine inactivation: - (i) by methylation of its imidazole ring which is effected by histamine-N-methyltransferase (HNMT); and (ii) by oxidative deamination of the primary amino group of histamine catalysed by diamine oxidase (DAO). HNMT is known to control the transmission of the histaminergic signals in the nervous system whereas DAO in serum or tissue-bound (e.g., intestinal mucosa, kidney) has been described responsible for degrading dietary histamine when passing through the mucosal into and in the circulation.

The present method comprises: - (i) administering a known amount of histamine labelled with a stable isotope and (ii) tracking any inactivated metabolites of isotopically labelled histamine by (iii) using a simple collective sample preparation of histamine and its polar metabolites and their subsequent analysis using a LC-MS/MS technique. An alternative to this method is by (a) adding isotopically labelled histamine to a blood, serum, or plasma sample from a patient; (b) incubating the spiked sample under physiological conditions for a predetermined time, and (c) having it analyzed using an LC-MS/MS technique after a simple collective sample preparation for small polar compounds.

The method of differential diagnosis of histamine intolerance syndrome comprises the following steps: (i) administering a subject suspected of suffering from insufficient histamine degradation activity a histamine load containing

a known amount of isotopically labelled histamine; (ii) obtaining a sample of bodily fluid selected from blood, serum, plasma, urine, or saliva after a predefined period of time; (iii) spiking of the said sample with a differently labelled internal standard; (vi) clean-up of the said spiked sample by protein precipitation and/or online or offline solid phase extraction; (v) measuring the stable isotope labelled products as well as the remaining stable-isotope-labelled histamine from the said sample; (vi) calculating a histamine conversion ratio or a conversion rate per unit time, based on the measured stable isotope-labelled fragments of methylhistamine, methylimidazole acetic acid, imidazole acetic acid and either the fragments of measured remaining stable isotopic-labelled histamine or the predefined concentration of used stable isotopic-labelled histamine, for comparison with the histamine conversion ratio and conversion rate found in a comparable sample from a healthy subject that has been administered with the same amount of stable isotopic-labelled histamine and diagnosis of histamine intolerance when said conversion is low.

By comparing the results on a single product basis, the method allows for a more detailed analysis of the single degradation and inactivation steps and allows directly to show which part of the histamine degradation process is impaired (HNMT, DAO). This provides the advantage to discriminate between the HMNT and DAO inactivation pathway as not only the reduction of histamine is measured and/or directly or indirectly the products of the DAO and/or HMNT degradation pathway; Furthermore, tissue bound DAO and other tissue bound degradation enzymes are considered too.

In some embodiments, the method may further comprise an immunological determination of the amount of soluble human diamine oxidase in serum or plasma.

The method of *in-vitro* diagnosis of histamine intolerance syndrome may comprise the steps of (i) obtaining a blood, plasma or serum sample from a human subject suspected of suffering from insufficient histamine inactivation activity; (ii) adding to said sample a predefined amount of *stable* isotope-labelled histamine to obtain a defined concentration in the sample; (iii) incubation of the said sample under physiological conditions for a defined time period to obtain *stable* isotope-labelled inactivation products; (iv) spiking of the said sample with a differently labelled internal standard; (vi) clean-up of the said spiked sample by protein precipitation and/or online or offline solid phase extraction; (v) measuring the *stable* isotope labelled products as well as the remaining *stable* isotope-labelled histamine from the said sample; (vi) calculating a histamine conversion ratio or a conversion rate per unit time, based on the measured *stable* isotope-labelled fragments of methylhistamine, methylimidazole acetic acid, imidazole acetic acid and either the fragments of measured histamine or the predefined concentration of used histamine, for comparison with the histamine conversion ratio and conversion rate found in a sample from a healthy subject and diagnosis of histamine intolerance when said conversion is low. By comparing the results on a single product basis, the method allows for a more detailed analysis of the single degradation steps and thus allows directly to show which part of the histamine degradation process is impaired (HNMT, DAO).

The method includes preferentially a spiking of the serum or plasma sample with an isotopically labelled histamine to achieve a spiking concentration from 10 nM (final concentration sample, e.g., histamine 1,84 ng/mL) to 50 μM litre-1 (final concentration sample, e.g., histamine 9200 ng/mL) blood, serum, or plasma, while a most preferred spiking concentration is 200 nM (histamine 36,8 ng /mL; final concentration in sample).

The method may also include a step of diluting the sample with an aqueous buffer containing 0,5 - 1,5 U/100 μl catalase/peroxidase and aldehyde dehydrogenase, more preferred 1 U/100 μl, followed by an incubation of the sample for 1 - 36 h at 30 - 40 °C and pH of 7.2 to 7.8, more preferably 6-24 h at 36 °C- 38 °C and pH 7.3 to 7.6.

For differential diagnosis, the method may comprise a calculating of the activity of soluble human DAO enzyme in said sample at given physiological conditions from the amounts of DAO substrates converted per unit of time and a determining of the half-life of histamine and/or DAO substrate in said sample, based on the measurement of imidazole acetic acid as well as stable isotope labelled histamine itself, to diagnose histamine intolerance when the patient's diamine oxidase activity is below 3 enzyme units (U) per millilitre serum or plasma or below 10 enzyme units (U) per millilitre as measured by the immunoassay. In some embodiments, the method further comprises an immunological determination of soluble human diamine oxidase in serum or plasma and a determination of the enzyme activity of DAO in serum or plasma.

In some embodiments, the method may comprise calculating the activity of tissue bound HNMT from a sample of bodily fluid taken from a subject that has been administered a known amount of stable isotope labelled histamine or a serum or plasma sample from a subject suffering from insufficient histamine inactivation enzyme activity spiked with a known amount of stable isotope labelled histamine based on the measured concentration of methylhistamine and/or methylimidazole acetic acid as well as the remaining histamine concentration from said samples and the elapsed incubation time. The calculated HNMT activity is than used for comparison with the corresponding histamine conversion ratio and conversion rate found in a sample from a healthy subject and ultimately for the diagnosis of histamine intolerance when said conversion is low.

In some embodiments, the method may comprise calculating of the total histamine clearance in plasma or serum in said sample at given physiological conditions from the total amounts of measured histamine inactivation products (methylhistamine, imidazole acetic acid, methylimidazole acetic acid) as well as the remaining stable isotope labelled histamine and used for diagnosis of histamine intolerance when the calculated histamine conversion ratio is low in

comparison to a healthy subject. Furthermore, the total histamine clearance can be used to evaluate potential anaesthesia-related risks, which can result from an increased histamine releasing in combination with an impaired histamine clearance capability of the subject.

In some embodiments, the method may comprise calculating of the total histamine clearance in the body of a subject after administration of a known concentration of stable isotope labelled histamine from the total amounts of measured histamine inactivation products (methylhistamine, imidazole acetic acid, methylimidazole acetic acid) as well as the remaining histamine measured in a collected plasma, serum, urine or salvia sample from said subject and used for diagnosis of histamine intolerance when the calculated histamine conversion ratio is low in comparison to a healthy subject. Again, the total histamine clearance can be used to evaluate potential anaesthesia-related risks, which can result from an increased histamine releasing in combination with an impaired histamine clearance capability of the subject.

Another aspect relates to a kit for *in-vitro* diagnosis of histamine intolerance syndrome, comprising a defined stable isotopic labelled histamine preparation as oral load, preferably in the form of a solution, dispersion or tablet, with isotopic labels as stated in formula I; internal standards for histamine labelled as stated in formula I, methylhistamine as stated in formula II, imidazole acetic acid as stated in formula III, methylimidazole acetic acid as stated in formula IV; suitable control and calibrator samples; as well as buffers and solutions for sample preparation and mobile phases for sample analysis.

Alternatively, the kit for *in-vitro* diagnosis of histamine intolerance syndrome may be one for measurement in a sample of plasma or serum. The histamine spiking solution or histamine load may contain a defined amount of stable isotopic labelled histamine labelled as stated in formula I; internal standards for histamine labelled as stated in formula I, methylhistamine as stated in formula II, imidazole acetic acid as stated in formula III, methylimidazole acetic acid as stated in formula IV; suitable control and calibrator samples; as well as buffers and solutions for sample preparation and mobile phases for sample analysis.

Another aspect relates to a differential *in-vitro* diagnosis of histamine intolerance syndrome comprising the steps of: (i) obtaining a blood, plasma or serum sample from a human subject suspected of suffering from insufficient histamine inactivation and/or DAO enzyme activity; (ii) determining the DAO concentration in that said sample using an immunoassay; (iii) adding to said sample a predefined amount of stable isotope-labelled DAO substrate, preferably histamine, to obtain a defined concentration in the sample; (iv) incubation of the said sample under physiological conditions for a defined time period to obtain stable isotopically labelled inactivation products; (v) spiking of the said sample with a differently labelled internal standard; (vi) clean-up of the said spiked sample by protein precipitation and/or online or offline solid phase extraction; (vii) measuring the stable isotope labelled products as well as the remaining isotopically labelled histamine from the said sample; (viii) calculating a histamine conversion ratio or a conversion rate per unit time, based on the measured isotopically labelled fragments of methylhistamine, methylimidazole acetic acid, imidazole acetic acid and either the fragments of measured histamine or the predefined concentration of used histamine, for comparison with the histamine conversion ratio and conversion rate found in a sample from a healthy subject and diagnosis of histamine intolerance when said conversion is low. By comparing the results on a single product basis, the method allows for a more detailed analysis of the single inactivation steps and may thus allow to directly show which part of the histamine inactivation process is impaired.

Symptoms of histamine intolerance usually appear only when the serum histamine level is higher than 9 nmol (about 1 ng /mL serum). However, it is not directly dependent on the amount of DAO in the blood or its activity. When foods and beverages contain up to 50 mg histamine per 100 grams, their consumption exceeds the physiologically available histamine clearance capacity. A DAO activity of 3 enzyme units in serum corresponds to a clearance of about 3 000 nmol histamine per minute. In patients with anaphylactoid reactions, pathological histamine concentrations of 5 to 10 000 nmol-liter-1 serum are observed. Thus, a DAO activity in serum of 3 enzyme units corresponds to a half-life of less than 2 minutes and to less than 4 hours for severely elevated histamine levels. Thus, a DAO activity in serum of 3 enzyme units or more rules out any symptoms of histamine intolerance. If the measured half-life of "spiked" (dietary) histamine in serum is below a threshold (120 seconds) the patient will likely not suffer from increased histamine levels, and this will also apply to a normal consumption of foods rich in histamine. However, we now know that a measurement of the statutory DAO or its activity in serum allows no differentiation between histamine intolerance and e.g., a food allergy. A conventional determination of a diamine/histamine conversion rate in serum allows no determination of the aetiology of histamine intolerance and why patients are suffering from an unphysiological histamine boost over an extended period (several hours or overnight).

The measured DAO activity following a spiking of a plasma or serum sample with a histamine challenge must not be confused with a measurement of the statutory DAO in serum as disclosed by prior art methods because the metabolic pathways of histamine and alcohol are linked. The primary metabolite of ethanol degradation, acetaldehyde, and the primary metabolites of histamine degradation, methylimidazole acetaldehyde and imidazole acetaldehyde, compete for the same aldehyde dehydrogenase and aldehyde oxidase. Alcohol and acetaldehyde further induce a release of histamine from the storage granules in peripheral mast cells so that a consumption of alcohol can provoke food-induced histaminosis which leads to gastric and intestinal damages and bronchial asthma in many persons. An increasing

epidemiological problem. Histamine levels in common alcoholic beverages range from 3 to 120 micrograms/L in white wine, 20 to 300 micrograms/L in beers, from 15 to 700 micrograms/L in champagnes and from 60 to 3800 micrograms/L in red wine.

The patient's complete capacity for histamine clearance (tissue associated DAO, secreted serum DAO and HNMT mediated) must be known for a differential diagnosis of histamine intolerance from food hypersensitivity, food intolerance and food allergy and this non-compromised (not inhibited by alcohol or drugs) total histamine clearance requires a measurement of all histamine catabolites in bodily fluids. This is because the amount and activity of tissue-associated DAO of a patient also contributes to the total dietary histamine clearance capacity. In the prior art, the serum DAO activity was never measured having regard to the total clearance of ingested histamine which involves tissue-associated DAO, and the histamine clearance by cellular HNMT has so far been ignored. The prior art methods merely determined which histamine depredation is achieved in the serum after addition of diamine (histamine or putrescine). In other words, the measurements were done with the expectation that the soluble DAO activity in serum would coincide with the total histamine clearance. That model did not consider the various histamine inactivation pathways nor the multiple forms of DAO nor the fact that the histamine clearance by the HNMT enzyme is ignored.

The present invention considers that the metabolic pathways may change by an excess of dietary histamine, that histamine inactivation in serum can be compromised by the presence of drugs or alcohol and that serum DAO activity is dependent on multiple factors, including pH because histamine may also be a substrate of monoamine oxidases (MAO) at higher pH above 8.0. Prior art assays even recommend using a Tris-HCl buffer (tris-(hydroxymethyl)-aminomethane) having a pH of 8.0 to 8.5 for a determining the diamine/histamine conversion ratio and rate as such a buffer solution has "particularly positive effects on the DAO activity" (see US 8 003 343, column 5). The conventional assays for diagnosis of histamine intolerance never examined or quantified the "inactivated" metabolites of histamine and could not differentiate between monoamine oxidase (MAO) activity and diamine oxidase (DAO) activity nor detect any inhibition, e.g., by external drugs or a lack of co-factors.

The prior art works with an excess of histamine or diamine in the assay and ignores the different Km values for histamine and putrescine so that no more than a maximal DAO activity at an artificial "pH and enzyme environment" is determined which is further compromised by competing metabolic pathways and varying amounts of histamine, diamine, DAO and/or MAO in the sample. Prior art solutions for measuring the DAO enzyme activity work with histamine or diamine in an amount of about 80 to 100 micromoles per litre, claiming a range from 5 to 400 micromoles, which is well above physiological levels. The usual limit for pathological histamine values in serum is 9 nanomoles per litre. Histamine concentrations in serum of patients with an anaphylactoid reactions are below 10 000 nanomoles (nmol) per litre (L), say less than 10 micromolar, with histamine disappearing from serum at 37°C with an apparent half-life of 2 minutes to 4 hours. Thus, a theoretical maximal DAO activity is determined or a wrong activity but not the actual DAO enzyme activity in serum. On the other hand, the present method allows an administration or oral administration of typical dietary or physiological concentrations of histamine due to the high sensitivity of the LC-MS/MS detection method. The resulting serum and urine levels of isotopic labelled imidazole acetic acid and methyl imidazole acetic acid, as well as of other histamine metabolites take account of the activity of tissue-associated DAO and HNMT, e.g., in the mucosal membrane and in the kidney, in addition to serum DAO.

The kinetic studies on the deaminating oxidation of $^{14}C$-putrescine at concentrations from 1 micromole to 5 millimole confirm the existence of two enzymes in serum: DAO with high affinity which is sensitive to alpha-aminoguanidine, and MAO with a lower affinity for the substrate which is selegiline-sensitive. The present disclosure refers to a method of diagnosing histamine intolerance when the diamine oxidase activity in serum is below 3 enzyme units (U) per millilitre serum, if determined *in vitro* at RT (25°C). The disclosure further comprises a method of diagnosis wherein the actual diamine oxidase activity in serum is 50% of the normalized diamine oxidase activity in serum as determined by an immunoassay for human DAO, indicating an inhibition of the enzyme DAO in serum by the presence of drugs or other biogenic amines.

The serum DAO concentration and the measured serum DAO activity by REA are already used biomarkers for histamine intolerance and associated symptoms. Both, serum DAO and the measured DAO activity in serum complement each other as the DAO activity in serum depends on the absence of inhibitors (drugs, alcohol) and cofactors such copper ions, manganese ions and vitamin C or vitamin B6. By further quantitating the cofactors in blood, the clinical laboratory will allow a treatment of the disorder in respect of the co-factors which have to be supplemented or which drug should be better replaced in order not to inhibit dietary histamine inactivation. A differential diagnosis of histamine intolerance syndrome vis-à-vis food intolerance, food hypersensitivity or food allergy is not possible.

Histamine intolerance (HIT) occurs when the combined activities of serum DAO; cellular HNMT, and tissue associated DAOs in the intestinal mucosal membrane, kidney, lungs are not enough to degrade a dietary histamine spike in addition to the histamine produced in the body. The overall histamine inactivation capacity in the body depends likewise on the absence of DAO inhibitors and the presence of co-factors. The role of HNMT, monoamino oxidases in the degradation and inactivation of consumed histamine also remains to be elucidated in detail. The large sequence identity between secreted DAO and tissue associated DAOs suggests that the inhibitors and co-factors affect most

similar the overall histamine inactivation capacity in the body as can be determined for the inactivation capacity of secreted serum DAO alone. The specific enzyme activity of DAO in a patient's sample is therefore a reference measure of the overall histamine inactivation ability in the body.

The specific enzyme activity is the amount of product formed in each amount of time under given conditions per milligram of enzyme. The specific activity is equal to the rate of reaction multiplied by the volume of reaction divided by the mass of total protein. The SI unit is the katal, 1 katal = 1 mol s-1, but this is an excessively large unit. A more commonly used value is the (one) enzyme unit (U) which is 1 micromole min-1. (One enzyme unit (U) corresponds to 16.67 nanokatals). The specific enzyme activity is therefore a measure of the processivity at a specific (usually saturating) substrate concentration. Where the molecular weight and the precise amount of the enzyme is not known, as in the present case, the measured turnover of isotope-labelled histamine in serum and the separately measured specific activity of serum DAO allow calculation of how much DAO enzyme, secreted or tissue-associated, is effectively present in the body and its total activity for inactivation of dietary histamine taken up with the foods. The turnover number can be visualized as the number of times each enzyme molecule conducts its catalytic cycle per second. The method of the invention therefore allows a reliable differentiation between histamine intolerance syndrome and food intolerance or food allergy. The histamine intolerance syndrome goes in line with an insufficient inactivation of a spike of dietary histamine for a lack of tissue-associated DAO in the intestinal mucosal membrane and to a minor degree for a lack of serum DAO. The symptoms of food allergy or food hypersensitivity are caused by a spike of endogenous histamine.

Gas chromatography-mass spectrometry (GC-MS) has been an analytical platform for detecting polar ketones, aldehydes, carboxylic acids because of its high resolution and sensitivity. The derivatization methods for GC analysis comprise a tandem oximation-silylation procedure. The oximation of aldehydes and ketones is achieved by a reaction between the carbonyl group and methoxyamine. The reaction with silylation agents replaces the active hydrogen in alcohols, carboxylic acids, amines, and thiols. Both reactions for a GC-based analysis are incompatibility with water. The necessary dehydration by solvent extraction, evaporation, or lyophilization makes the sample preparation for GC-MS analysis time-consuming and the entire process is therefore too inefficient for a use in a clinical laboratory. Thus, a method for liquid chromatography-tandem mass spectrometry (LC-MS/MS) was developed which is compatible with bodily fluids and thus allows for a faster and easier sample preparation. A direct LC-MS/MS analysis of histamine and its degradation/inactivation products of histamine is difficult due to their poor chromatographic performance and low ionization efficiency. The reverse phase LC column seemed not well suited for retaining and separating these hydrophilic metabolites. The present inventors however found out that the hydrophilic interaction liquid chromatography (HILIC) offers sufficient chromatographic separation of polar compounds as well as a sufficient detectability of the histamine degradation products: imidazole acetic acid and N-methylimidazole acetic acid.

The isotopic labelled histamine may be labelled at positions shown in formulae I. Preferred internal standards of the expected histamine metabolites may be labelled additionally, e.g. at least at one more position, preferably at three or four positions with the corresponding heavy isotope.

As shown in Fig. 1, histamine is inactivated intracellularly by histamine N-methyltransferase (NMT) and extracellularly by diamine oxidase (DAO) and enzymes of the monoamine oxidase family (MAO). The histamine inactivation pathways all merge - irrespective of the oxidation by secreted DAO or membrane-associated DAO or any MAO - at compounds of formulae II and III (imidazole-4-acetic acid and N-methyl-imidazole-4-acetic acid). The disclosed direct determination of histamine catabolites allows examination of all histamine inactivation pathways and allows holistic diagnosis. More precisely, the described method allows to follow up and detect simultaneously all inactivation products of histamine (His), including the initial methylation to N-methyl histamine (MetHis) and the subsequent oxidation to N-methyl imidazole acetic acid (Mimi) of the inactivation/degradation pathway (1) as well as the extracellular inactivation and oxidative degradation to imidazole acetic acid (Imi) of degradation pathway (2). The two imidazole acetic acid products of isotopic labelled histamine as well as histamine and methyl histamine can be separated using hydrophilic interaction chromatography (HILIC) coupled to mass spectrometry; see Fig. 2. The aprotic solvent may be selected from acetonitrile, tetrahydrofuran, acrylonitrile, dioxane, ethanol but acetonitrile is a preferred solvent because it works equally with zwitterionic HILIC LC columns, PEI HILIC LC columns, silica HILIC columns and urea HILIC columns, as well as with amide HILIC columns and other reversed phase columns. In HILIC, the mobile phase forms a water-rich layer on the surface of the polar stationary phase versus the water-deficient mobile phase, creating a liquid/liquid extraction system. The analytes (histamine, methylhistamine, imidazole acetic acid and methyl imidazole acetic acid) are therefore distributed between these two layers. HILIC should be considered more than a simple partitioning because of its hydrogen donor interactions between neutral polar species as well as weak electrostatic mechanisms under the high organic solvent conditions. These are used for retention and distinguishes HILIC from ion exchange chromatography. The more polar compounds have a stronger interaction with the stationary aqueous layer than the less polar compounds. Thus, a separation based on a compound's polarity and degree of solvation takes place and for this acetonitrile is a well-suited mobile phase.

The Lower Limit Of Quantitation (LLOQ) of the disclosed method and with respect to the described analytes ((histamine, methyl histamine, imidazole acetic acid and methyl imidazole acetic acid) is in the range of 100 pg/mL

(picograms) and consequently sufficiently sensitive for all purposes of diagnosis.

The present application discloses that the inactivation products of histamine - imidazole-4-acetic acid and methyl imidazole-4- acetic acid can also be separated on a HILIC column when acetonitrile is used as a mobile phase. The combination of the LC-MS/MS technique, the use of an isotopic labelled histamine for an oral histamine load and/or for spiking a blood, serum or plasma sample, and the analytical sensitivity for the histamine inactivation products, this combination allows a differential diagnosis of histamine intolerance syndrome and compared to anaphylactoid and allergic reactions. The alternative clinical laboratory method may comprise the steps: (i) taking an aliquot of blood, plasma or serum from a patient suspected of suffering from histamine intolerance syndrome; (ii) optionally, determining the concentration of human diamine oxidase (hDAO - EC 1.4.3.22) in said aliquot using an immunoassay for human diamine oxidase (DAO); (iii) mixing (spiking) an aliquot with a predefined amount of isotopic labelled histamine of formula I at a concentration range of from 10 to 10 000 nanomoles/L, preferably from 100 to 500 nanomoles/L and incubation of the histamine spiked sample at physiological conditions (pH 7.2 to 7.8, preferably 7.3 to 7.6; 36-38°C) for a predefined period from 2 minutes to 24 hours; (iv) adding a suitable internal standard to the said sample (v) precipitate proteins and inorganic salts from the sample with an aprotic solvent, preferably acetonitrile (vi) transferring said aliquot to a mass spectrometry device, preferably a device comprising a combination of HILIC liquid chromatography and tandem mass spectrometry and (vii) determining by mass spectrometry in said aliquot the concentrations of histamine inactivation products, and optionally of histamine, to obtain a histamine inactivation ratio and a histamine inactivation rate; to diagnose a histamine intolerance when the patient's oxidative inactivation activity in said sample is below a threshold level. This threshold level may be set at 3 enzyme units (U) per milliliter serum at physiological conditions of the blood and/or normalized 10 enzyme units (U) per millilitre; or a half-life of dietary histamine levels of above a certain threshold for healthy subjects (2 minutes to 24 hours, depending on concentrations).

A person skilled in the art will also contemplate to directly determine the histamine concentration in the plasma sample to determine whether it is in a pathological range or the inactivation of histamine to pathway (2) which according to medical literature does not take place in serum.

The isotopic labelled histamine used for oral administration, incubation or internal standard may contain $^{13}C$ and/or $^{15}N$ and/or D at one or multiple positions as shown in the formula I.

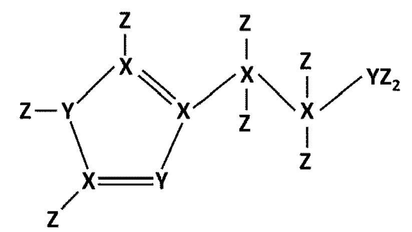

X = $^{13}C$ Y = $^{15}N$ Z = $^{2}H$ (D)

Formula I (histamine)

Depending on the utilized isotopic labelled histamine the resulting stable isotope labelled products will be methylhistamine (formula II), imidazole acetic acid (formula III) and methylimidazole acetic acid (formula IV). Furthermore, formulae I-IV give reference for labelling possibilities for $^{13}C$, $^{15}N$ and D labelling for corresponding internal standards:

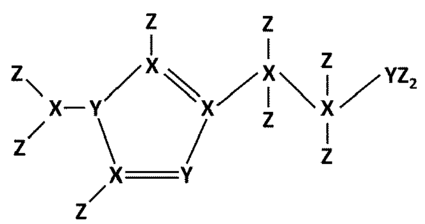

X = $^{13}C$ Y = $^{15}N$ Z = $^{2}H$ (D)

Formula II (methylhistamine)

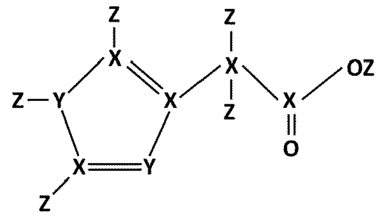

$X = {}^{13}C$  $Y = {}^{15}N$  $Z = {}^{2}H$ (D)

Formula III (imidazole acetic acid)

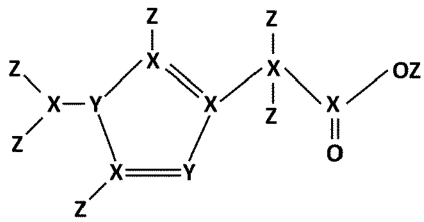

$X = {}^{13}C$  $Y = {}^{15}N$  $Z = {}^{2}H$ (D)

Formula IV (methylimidazole acetic acid)

In case of isotopic labelled histamine, the intact molecule and possible fragmentation products are shown and the corresponding full scan spectrum of fragmented unlabelled histamine *(m/z* 112,117 $[M+H]^+$) is shown in **Fig. 4.**

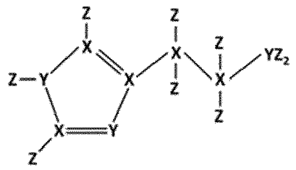

$X = {}^{13}C$  $Y = {}^{15}N$  $Z = {}^{2}H$ (D)

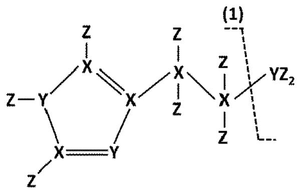

$X = {}^{13}C$  $Y = {}^{15}N$  $Z = {}^{2}H$ (D)

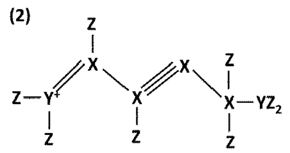

$X = {}^{13}C$  $Y = {}^{15}N$  $Z = {}^{2}H$ (D)

The intact molecule and fragmentation products of ***d4*** labelled histamine *(m/z* 116,133 $[M+H]^+$):-

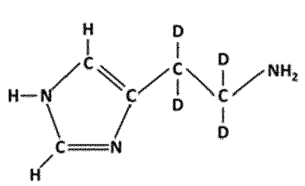

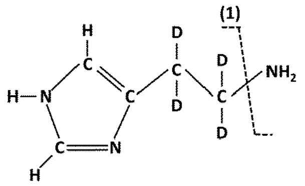

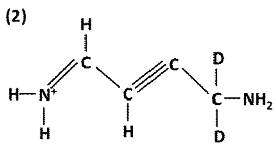

and a representative full scan mass spectrum of fragmented ***d4*** labelled histamine is shown in Fig. 5.

In case of ***$^{13}$C5*** labelled histamine *(m/z* 117,113 $[M+H]^+$), the intact molecule and possible fragmentation products are:-

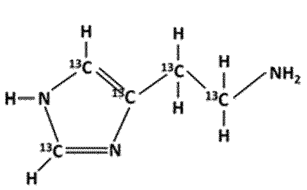

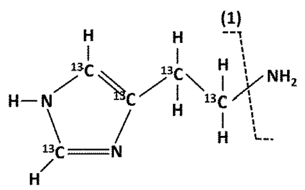

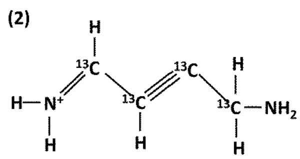

and **Fig. 6** shows a representative full scan mass spectrum of the possible fragment products of ***$^{13}$C5*** labelled histamine.

In case of isotopically labelled methyl histamine, the intact molecule and possible fragmentation products are shown below:

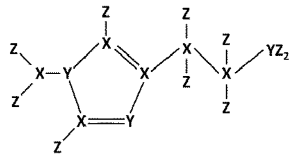

X = ¹³C Y = ¹⁵N Z = ²H (D)

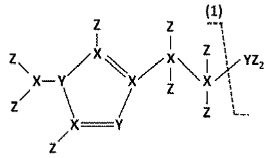

X = ¹³C Y = ¹⁵N Z = ²H (D)

(2)

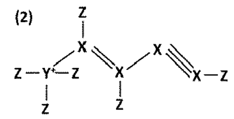

X = ¹³C Y = ¹⁵N Z = ²H (D)

and **Fig. 7** shows a representative full scan mass spectrum of fragmented unlabelled histamine *(m/z* 126,128 $[M+H]^+$).

With respect to $^{13}$***C5*** methyl histamine *(m/z* 131,128 $[M+H]^+$), the intact molecule and possible fragments are shown below:

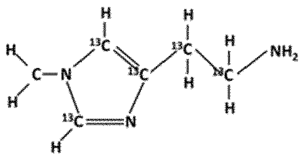

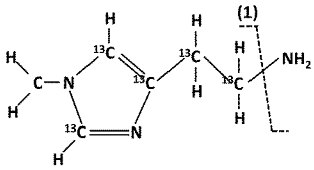

(2)

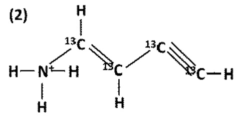

and **Fig. 8** shows a full scan spectrum of fragmented $^{13}$***C5*** methyl histamine.

With respect to isotope labelled imidazole acetic acid, the intact molecule and possible fragmentation structures are shown below

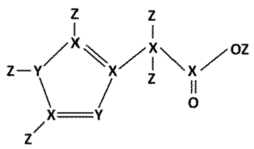

X = ¹³C Y = ¹⁵N Z = ²H (D)

(1)

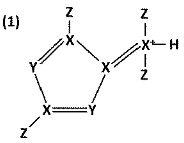

X = ¹³C Y = ¹⁵N Z = ²H (D)

(2)

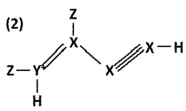

X = ¹³C Y = ¹⁵N Z = ²H (D)

(3)

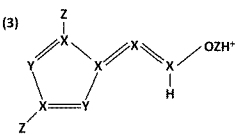

X = ¹³C Y = ¹⁵N Z = ²H (D)

and **Fig. 9** shows a full scan spectrum of fragmented imidazole acetic acid *(m/z* 127,069 $[M+H]^+$).

With respect to $^{13}$***C5*** imidazole acetic acid *(m/z* 132,073 $[M+H]^+$), the intact molecule and its possible fragments are shown below:

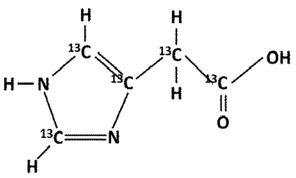

(1)

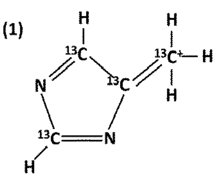

(2)

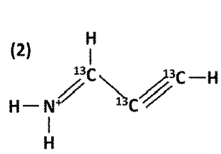

(3)

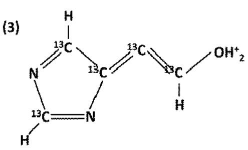

and a full scan MS/MS spectrum for analysis is shown in **Fig. 10.**

In case of isotopic labelled methyl imidazole acetic acid, possible fragmentation structures are as shown below:

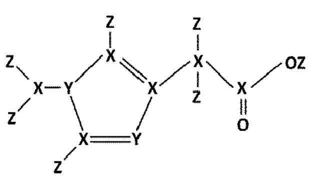

X = ¹³C Y = ¹⁵N Z = ²H (D)

(1)

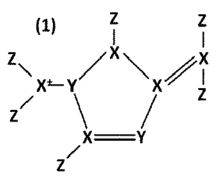

X = ¹³C Y = ¹⁵N Z = ²H (D)

(2)

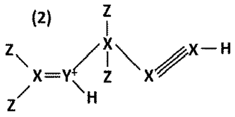

(3)

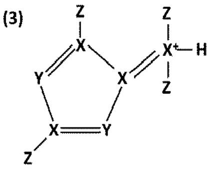

X = $^{13}C$ Y = $^{15}N$ Z = $^{2}H$ (D) X = $^{13}C$ Y = $^{15}N$ Z = $^{2}H$ (D)

and **Fig. 11** shows a full scan spectrum of fragmented unlabelled methylimidazole acetic acid *(m/z* 141,093 [M+H]$^+$).

In case of $^{13}$***C5*** methyl imidazole acetic acid *(m/z* 146,088 [M+H]$^+$), the intact molecule and its possible fragments are shown below :

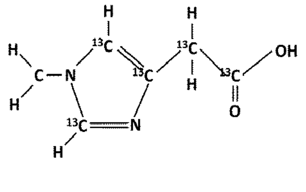

(1)

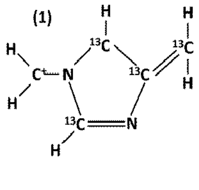

(2)

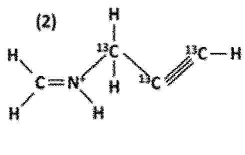

(3)

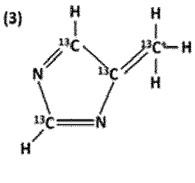

and a full scan MS/MS spectrum thereof is shown in Fig. 12.

<u>EXAMPLES</u>

***Example 1:*** *- Isotopic labelled catabolites of histamine and their determination by HILIC LC-MS/MS*

900 μl serum sample from a patient with suspected HIT (no prior provocation) was placed in a 1,5 ml reaction tube and incubated at 37 °C with 100 μl aqueous buffer containing 1 μM (184 ng/mL) isotopic labelled histamine and 1 U/100 μl catalase/peroxidase and aldehyde dehydrogenase for predefined periods of time (2h, 4h, 6h, 24h).

The patient with suspected HIT was also given an oral solution of isotopic labelled histamine to achieve histamine provocation. The histamine provocation must be adjusted to the patient. The provocation can be in the range of 0,01 mg/kg and 0,1 mg/kg, less preferably 0,001 mg/kg and 0,5 mg/kg. Thereafter, urine and serum sample were collected at regular intervals, preferably between 1 and 6 hours, more preferably between 2 and 4 hours.

50 μl of the sample (serum, plasma, urine; independent of sample collection) were added to a reaction vessel and mixed with 10 μl of internal standard solution (*aqua bidest*). The concentrations of the internal standards can be between 1 - 100 ng/ml, preferably between 10 - 50 ng/ml, most preferably between 20-30 ng/ml. The sample was then mixed with 1140 μl acetonitrile and vortexed vigorously for 30 seconds.

The mixed samples were incubated at -20 °C for 1 hour, again shaken for 30 seconds and centrifuged at 14000 rpm at 4 °C for 10 minutes.

The supernatants were transferred directly to autosampler vials and fed to the LC-MS/MS system or dried in a rotary vacuum evaporator until dryness. The dried samples can be stored at -20° and reconstituted with 100 μl reconstitution solution consisting of 5-20 % *aqua bidest* in acetonitrile, preferably 5-10 % *aqua bidest* in acetonitrile.

Baseline separation of all analytes (isotopic labelled histamine and all its catabolites) was performed by a HILIC (hydrophilic interaction liquid chromatography) column from Agilent (InfinityLab Poroshell 120 HILIC-Z - 150 x 2.1 mm, 2.7 μm particle size). Similar HILIC columns or shorter versions of the column may be used but have not been tested. The sample was separated using two mobile phases; mobile phase A consisted of 5% acetonitrile at 95% in water with 10 mM ammonium formate at pH 3; mobile phase B consisted of 85% acetonitrile in water with 10 mM ammonium formate at pH 3. The flow rate was 0.5 ml/min and the column operated at 25°C. The gradient consisted of a loading step of 30 seconds at 100% B; an increase from 100% B to 60% B over 3 minutes; a constant part at 60% B for 30 seconds; an increase from 60% to 100% in 30 seconds; a re-equilibration phase at 100% B for 3.5 minutes (see **Fig. 2).** A skilled person will optimize the gradient and mobile phase according to the utilized column and LC system, and thus result in shorter gradients time with the same level of chromatographic resolution. **Fig. 3** shows a representative chromatogram of the retention (elution) of histamine and its catabolites (methylhistamine, imidazole acetic acid and methyl imidazole acetic acid) from said spiked and analysed serum sample.

**Fig. 4** depicts a labelled histamine with two possible fragment structures. The following fragments from **Table 1** were used as qualifiers/quantifiers for labelled histamine and e.g. ***d4*** and $^{13}C_5$ stable isotopic labelled histamine. **Fig. 5** shows a representative full scan spectrum of fragmented unlabelled histamine *(m/z* 112,117 [M+H]$^+$) and its fragments and proposed structures. **Fig. 6** then shows a representative full scan spectrum of deuterium labelled (***d4***) histamine (*m/z* 116, 133 [M+H]$^+$) and its fragments and possible structures. **Fig. 7** then shows a representative full scan spectrum

of *$^{13}C_5$* labelled histamine (*m/z* 117,133 [M+H]+) .

TABLE 1

| Fragments used as qualifier/quantifier for the identification of unlabelled, d4 and 13C5 labelled histamine. | | | |
|---|---|---|---|
| **Analyte** | **Precursor $[M+H]^+$** | **Fragment 1 $[M+H]^+$** | **Fragment 2 $[M+H]^+$** |
| Histamine | 112,117 | 95.19 | 81.20 |
| Histamine *D4* | 116,133 | 99.21 | 85.20 |
| Histamine $^{13}C_5$ | 117,133 | 100.20 | 87.20 |

The following exemplary fragments from Table 2 were used as qualifiers/quantifiers for methylhistamine and *$^{13}C_5$* labelled methylhistamine.

TABLE 2

| Fragments used as qualifier/quantifier for the identification of unlabelled and 13C5 labelled methylhistamine. | | | |
|---|---|---|---|
| Analyte | Precursor [M+H]+ | Fragment 1 [M+H]+ | Fragment 2 [M+H]+ |
| Methylhistamine | 126.128 | 109.21 | 68.23 |
| Methylhistamine 13C5 | 131.138 | 114.22 | 71.2 |

**Fig. 8** depicts a labelled methylhistamine with two possible fragment structures. **Fig. 9** shows a representative full scan spectrum of fragmented labelled methylhistamine (*m/z* 126,128 $[M+H]^+$), intact molecule and possible fragment structures and **Fig. 10** a representative full scan spectrum of fragmented *$^{13}C_5$* methylhistamine (*m/z* 131,128 $[M+H]^+$), intact molecule and possible fragment structures.

The following exemplary fragments from Table 3 were used as qualifier/quantifier for imidazole acetic acid. Fragmentation of a labelled imidazole acetic acid.

TABLE 3

| *Fragments used as qualifier/quantifier for the identification of labelled and $^{13}C_5$ imidazole acetic acid.* | | | | |
|---|---|---|---|---|
| **Analyte** | **Precursor $[M+H]^+$** | **Fragment 1 $[M+H]^+$** | **Fragment 2 $[M+H]^+$** | **Fragment 3 $[M+H]^+$** |
| Imidazole acetic acid | 127.069 | 81.18 | 54.2 | 109.18 |
| Imidazole acetic acid $^{13}C_5$ | 132.073 | 85.2 | 57.21 | 114.2 |

**Fig. 11** depicts a theoretically labelled imidazole acetic acid with three possible fragment structures. **Fig. 12** shows a representative full scan spectrum of fragmented labelled imidazole acetic acid *(m/z* 127,069 $[M+H]^+$), intact molecule and possible fragment structures and **Fig. 13** depicts a representative full scan spectrum of fragmented *$^{13}C_5$* imidazole acetic acid *(m/z* 132,073 $[M+H]^+$), intact molecule and possible fragment structures.

The following exemplary fragments from Table 4 were used as qualifier/quantifier for methylimidazole acetic acid.

TABLE 4

| *Fragments used as qualifier/quantifier for the identification of labelled and $^{13}C_5$ labelled methylimidazole acetic acid.* | | | | |
|---|---|---|---|---|
| **Analyte** | **Precursor $[M+H]^+$** | **Fragment 1 $[M+H]^+$** | **Fragment 2 $[M+H]^+$** | **Fragment 3 $[M+H]^+$** |
| methylimidazole acetic acid | 141.083 | 95.02 | 68.09 | 81.18 |
| methylimidazole acetic acid $^{13}C_5$ | 146.088 | 99.16 | 71.10 | 85.10 |

**Fig. 11** shows a representative full scan spectrum of fragmented unlabelled methylimidazole acetic acid (m/z 141,093 $[M+H]^+$), intact molecule and possible fragment structures and **Fig. 12** depicts a representative full scan spectrum of fragmented *$^{13}C_5$* labelled methylimidazole acetic acid *(m/z* 146,088 $[M+H]^+$), intact molecule and possible fragment

structures.

The skilled person may choose also another isotope labelling for unambiguous detection by LC-MS/MS for any of the analytes to increase the sensitivity of the method which is in range of 100 pg per any analyte which depends also on the employed mass spectrometry. A skilled person will also appreciate that the isotopically labelling of the internal standards may be the same or different to the stable isotope labelling of histamine. Preferred is having a 13C labelled substrate and deuterium labelled standard or vice versa.

## Claims

**1.** A method of diagnosis of histamine intolerance syndrome in a human subject suspected of suffering from insufficient total histamine inactivation activity, comprising:-

administering a preparation, solution, or suspension containing a known amount of histamine, labelled by a stable isotope;
obtaining samples of bodily fluids selected from blood, serum, plasma, urine, or saliva after one or more predetermined periods of time;
facultatively, adding an aqueous buffer containing stable isotope labelled internal standards;
adding an aprotic solvent miscible with the aqueous sample to the samples and removing proteins and organic salts from the samples;
determining the amounts of isotopically labelled histamine in said sample and one or more other isotopically labelled inactivation products of histamine using hydrophilic interaction chromatography and mass spectrometry which comprises an LC-MS/MS technique; and
calculating the histamine inactivation activity of the subject on basis of the amounts of isotopically labelled histamine and imidazole acetic acid and comparing the found histamine inactivation activity with the histamine inactivation activity found in healthy subjects.

**2.** The method of claim 1, further comprising a determination of isotopically labelled methyl imidazole acetic acid in the sample.

**3.** The method of claim 1 or claim 2, further comprising a determination of isotopically labelled methylhistamine in the sample.

**4.** The method of claim 1 or claim 2, comprising an immunological determination of secreted human diamine oxidase in serum or plasma.

**5.** The method of any preceding claim 1 to 4, wherein the isotopically labelled histamine is selected from histamine $^{15}$***N***-labelled at positions at anyone or more nitrogen position or a histamine $^{13}$*C*-labelled at anyone or more carbon positions or a histamine deuterium labelled at anyone or more hydrogen position, or a histamine containing a combination of stable isotope labels.

**6.** The method of any preceding claim 1 to 6, wherein the aprotic solvent is selected from acetonitrile, tetrahydrofuran, acrylonitrile, dioxane, ethanol, methanol preferably from acetonitrile.

**7.** The method of any preceding claim 1 to 7, comprising a determination of any one of the isotope labelled ratios selected from relative amounts of

histamine / methylhistamine,
histamine / imidazole acetic acid,
methyl histamine / methyl imidazole acetic acid;
histamine /

methylhistamine + imidazole acetic acid + methyl imidazole acetic acid,

and optionally, a determining which route of histamine inactivation is inhibited or deficient.

**8.** A method of diagnosis of histamine intolerance syndrome in a subject suspected of suffering from insufficient secreted

DAO activity and/or deficient HNMT activity, comprising the steps of:-

obtaining a sample of plasma or serum from said subject suspected of suffering from histamine intolerance;
adding to said sample a predefined amount of stable isotope-labelled histamine to produce a defined concentration of isotopically labelled histamine in said sample, wherein the stable isotopic labelled histamine is selected from histamine $^{15}N$-labelled at positions at anyone or more nitrogen position or a histamine $^{13}C$-labelled at anyone or more carbon positions or a histamine deuterium labelled at anyone or more hydrogen position, or a histamine containing a combination of stable isotope labels;
incubation of said sample for a predefined period under physiological conditions to obtain inactivation of said isotopically labelled substrate by the activity of DAO and/or enzyme contained in said sample of plasma or serum;
adding an aqueous buffer containing stable isotope labelled internal standards;
adding an aprotic solvent miscible with the aqueous sample to the samples, or a predetermined aliquot thereof, and removing proteins and organic salts from the samples;
determining the amounts of isotopically labelled histamine in said sample and one or more other isotopically labelled inactivation products of histamine using hydrophilic interaction chromatography and mass spectrometry which comprises an LC-MS/MS technique; and
calculating the histamine inactivation activity and/or histamine inactivation activity and/or the DAO activity in plasma or serum of the subject on basis of the amounts of isotopically labelled histamine, methyl histamine and it catabolites with imidazole acetic acid.

**9.** The method of claim 7, comprising a spiking of the sample with an isotopically labelled histamine to achieve a spiked-histamine concentration of from 200 to 500 nmol·litre-1 serum.

**10.** The method of preceding claim 7 or claim 8, further calculating the activity of soluble human DAO enzyme and HNMT enzyme in said sample at given physiological conditions from the amounts of isotopically labelled histamine substrate converted per unit of time and determining the histamine inactivation or half-life of histamine in said patient, to diagnose a histamine intolerance when the patient’s diamine oxidase activity is below 3 enzyme units (U) per milliliter serum or plasma or below 10 enzyme units (U) per milliliter as measured by the immunoassay and/or the half-life of stable isotopically labelled histamine in serum is above a threshold found in a sample of a healthy subject.

**11.** The method of any preceding claim 7 to 9, comprising an immunological determination of soluble human diamine oxidase in serum or plasma and a determination of the enzyme activity of DAO in serum or plasma.

**12.** A kit for diagnosis of histamine intolerance syndrome as claimed in any claim 1 to 6, comprising:-

a defined histamine oral load in the form of a solution, dispersion or tablet, which contains a defined amount of stable isotopically labelled histamine selected from histamine $^{15}$N-labelled at positions N1 and/or N3 or a histamine $^{13}$C-labelled at positions C2, C4, C5, C6, or C7 or a histamine deuterium labelled at one or more hydrogen positions; and
and solutions containing stable isotope labelled standards of the respective catabolites, selected from proposed isotopically labelled positions of histamine, imidazole acetic acid, methyl histamine and methyl imidazole acetic.

**13.** A kit for diagnosis of histamine intolerance syndrome in a sample of plasma or serum as claimed in any claim 7 to 9, comprising:-

a histamine spiking solution, which contains a defined amount of stable isotopically labelled histamine selected from histamine $^{15}$N-labelled at positions N1 and/or N3 or a histamine $^{13}$C-labelled at positions C2, C4, C5, C6, or C7 or a histamine deuterium labelled at anyone or more hydrogen position, or a histamine having a combination of stable isotope labels; and
and solutions containing stable isotope labelled standards, selected from proposed isotope labelled positions of histamine, imidazole acetic acid, methyl histamine and methyl imidazole acetic.

**14.** A kit as claimed in claim 12, comprising an enzyme solution of catalase, peroxidase, and/or aldehyde dehydrogenase.

**15.** A kit as claimed in claim 12, comprising an enzyme solution of catalase, peroxidase, and/or aldehyde dehydrogenase.

**Fig. 1**

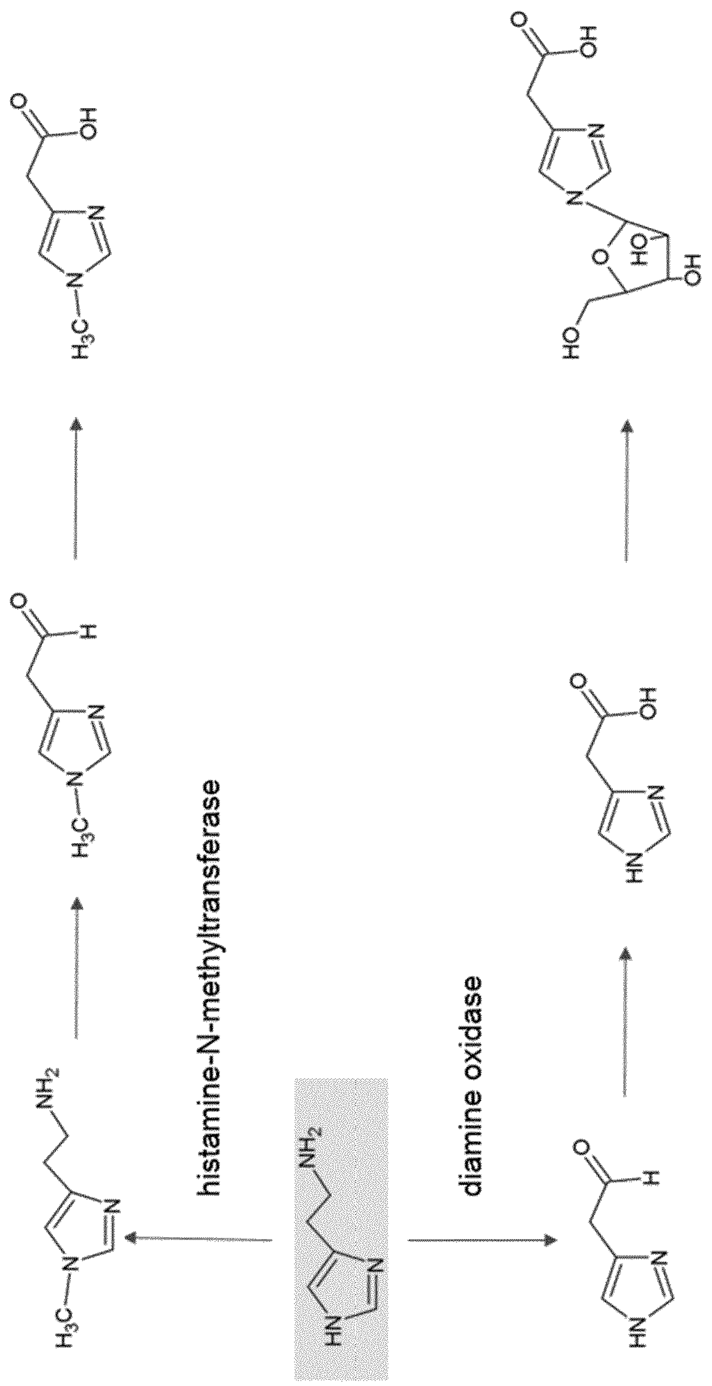

# Fig. 2

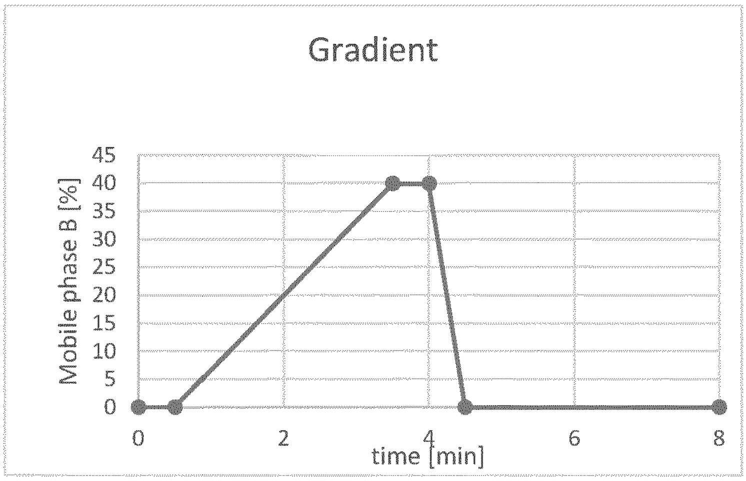


| Time [min] | B [%] |
|---|---|
| 0 | 0 |
| 0,5 | 0 |
| 3,5 | 40 |
| 4 | 40 |
| 4,5 | 0 |
| 8 | 0 |

# Fig. 3

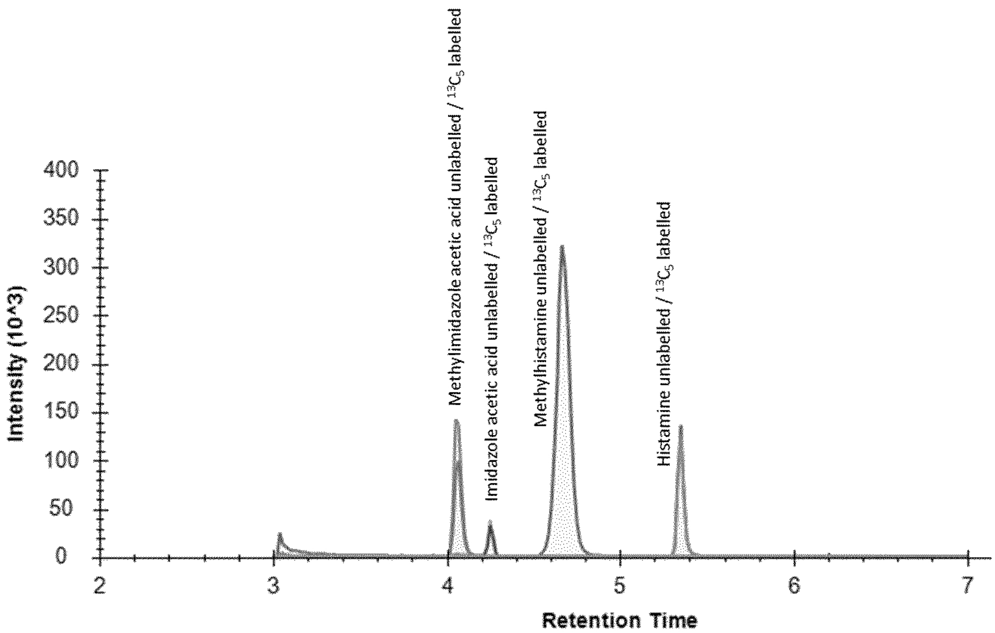

# Fig. 4

*Full scan spectrum of fragmented unlabelled histamine (m/z 112,117 [M+H]+)*

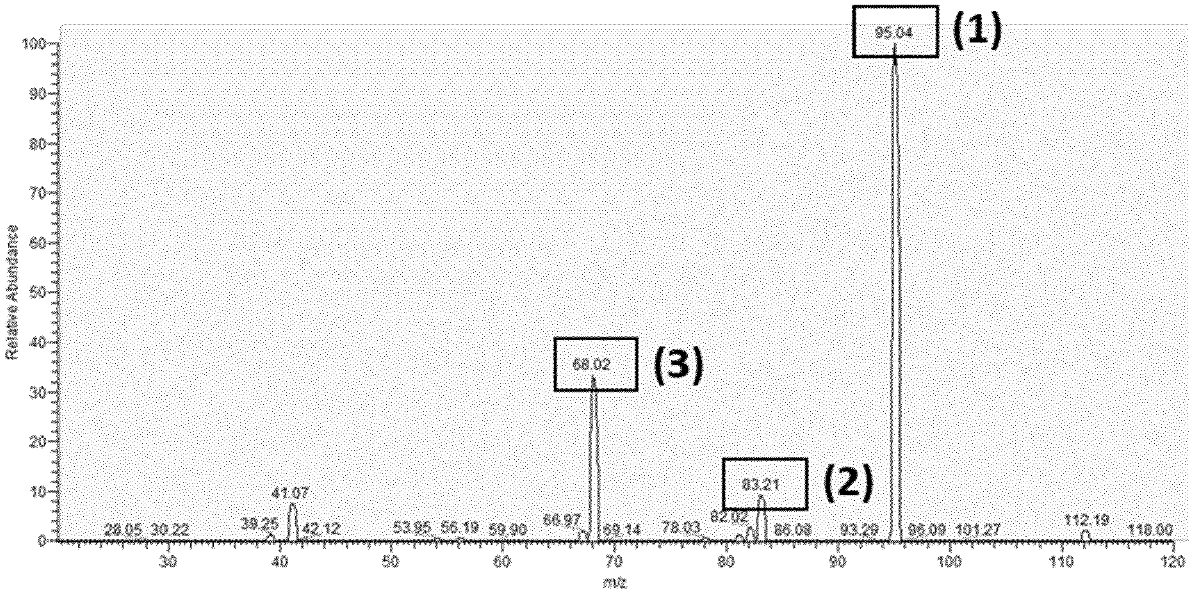


# Fig. 5

*Full scan spectrum of fragmented d4 labelled histamine (m/z 116,133 $[M+H]^+$)*

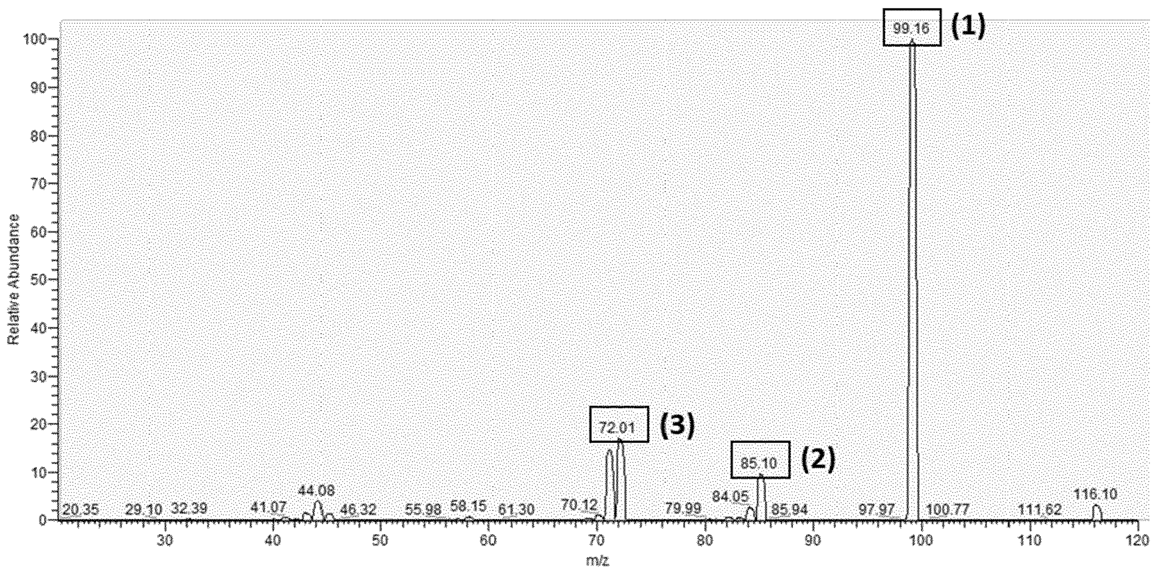

# Fig. 6

*Full scan spectrum of fragmented $^{13}C_5$ labelled histamine (m/z 117,113 $[M+H]^+$)*

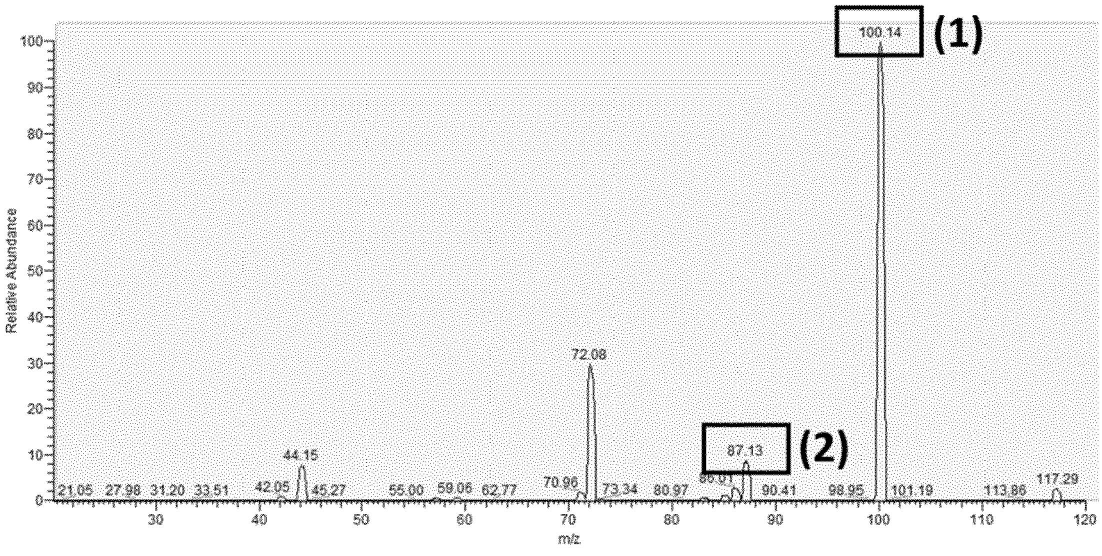


# Fig. 7

*Full scan spectrum of fragmented unlabelled methylhistamine (m/z 126,128 [M+H]+)*

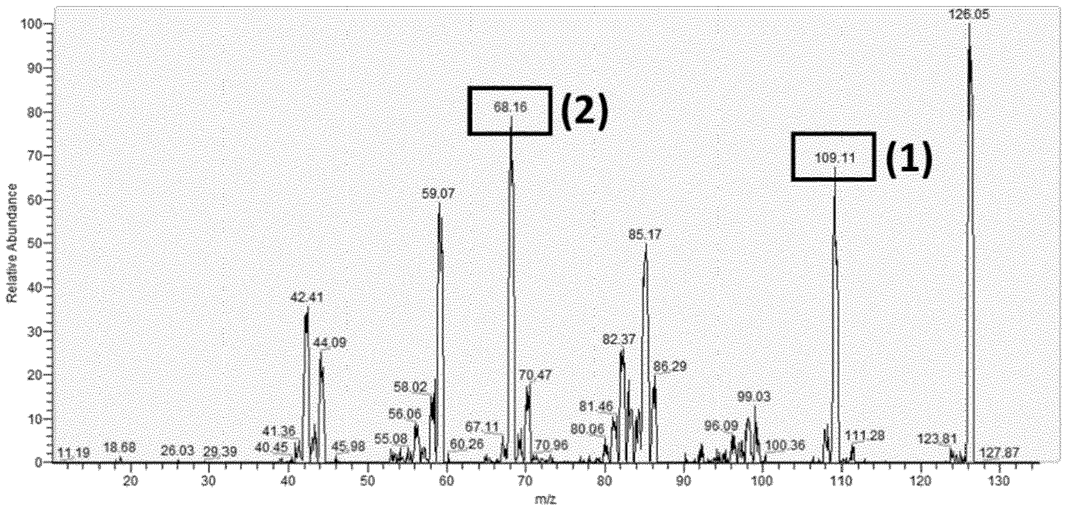

# Fig. 8

*Full scan spectrum of fragmented $^{13}C_5$ methylhistamine (m/z 131,128 [M+H]+)*

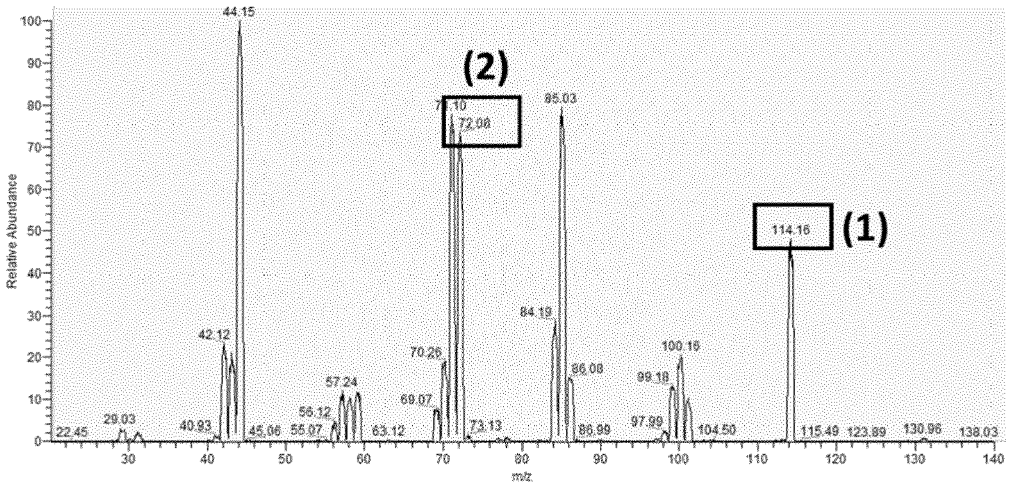


# Fig. 9

*Full scan spectrum of fragmented unlabelled imidazole acetic acid (m/z 127,069 [M+H]+)*

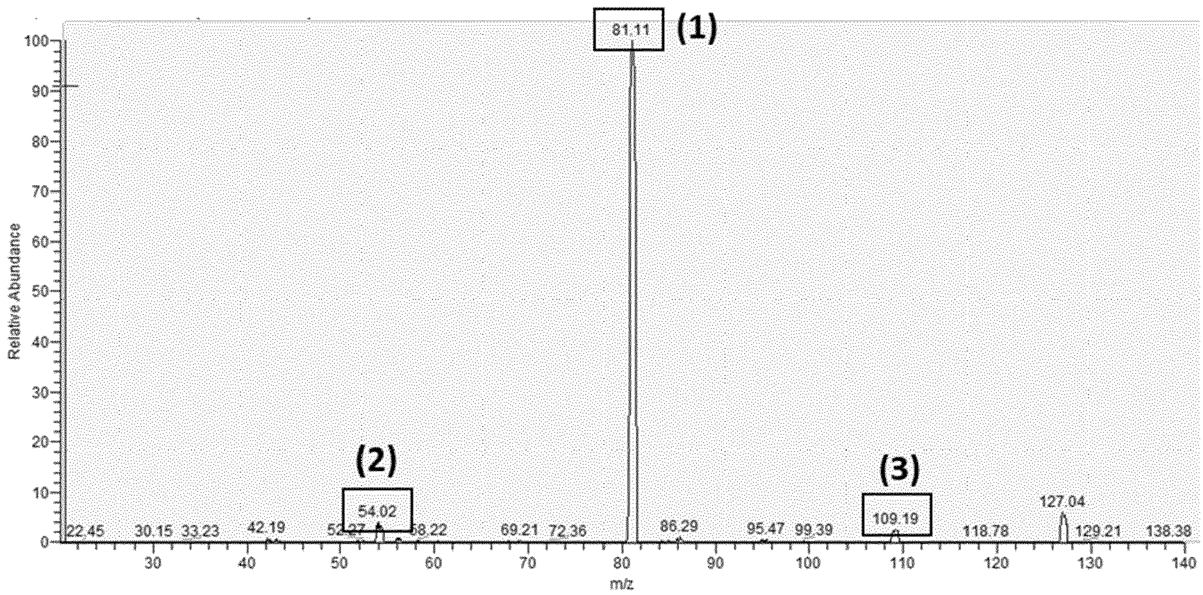

# Fig. 10

*Full scan spectrum of fragmented $^{13}C_5$ labelled imidazole acetic acid (m/z 132,073 $[M+H]^+$)*

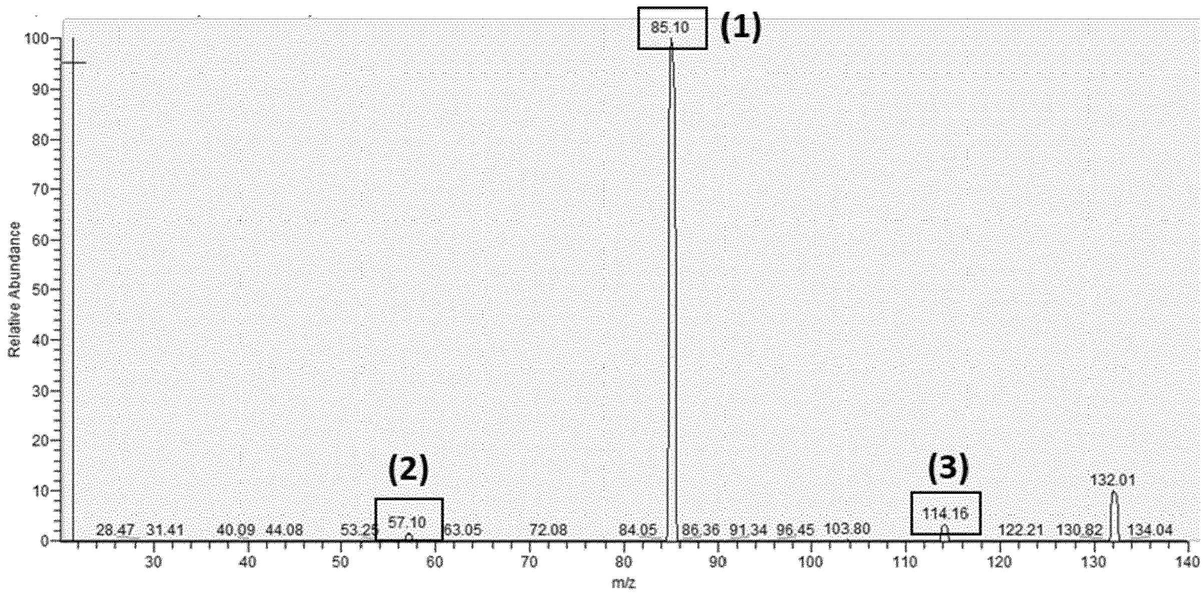


# Fig. 11

*Full scan spectrum of fragmented unlabelled methyl imidazole acetic acid (m/z 141,093 [M+H]+)*

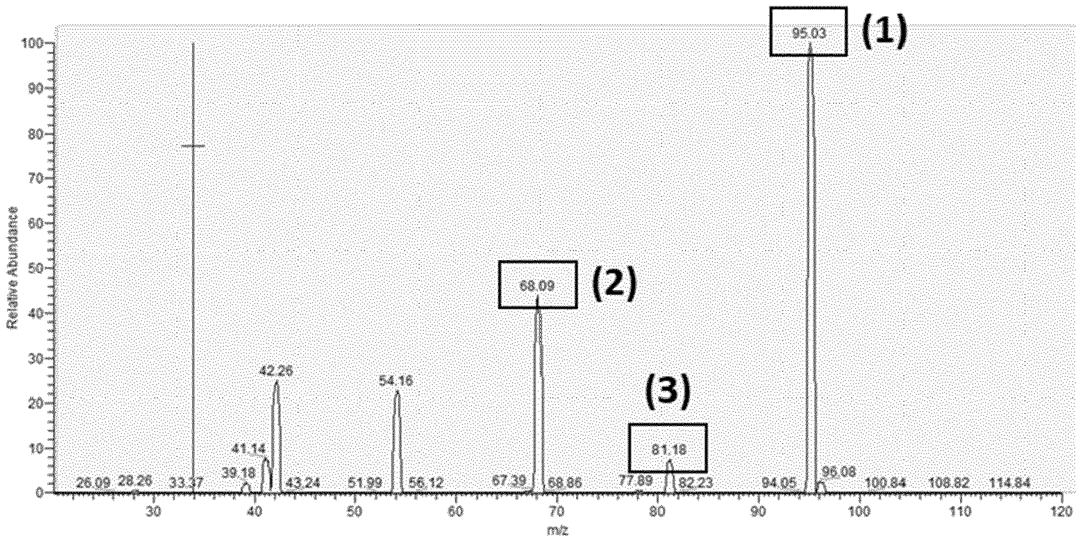

# Fig. 12

*Full scan spectrum of fragmented 13C5 labelled methyl imidazole acetic acid (m/z 146,088 [M+H]+)*

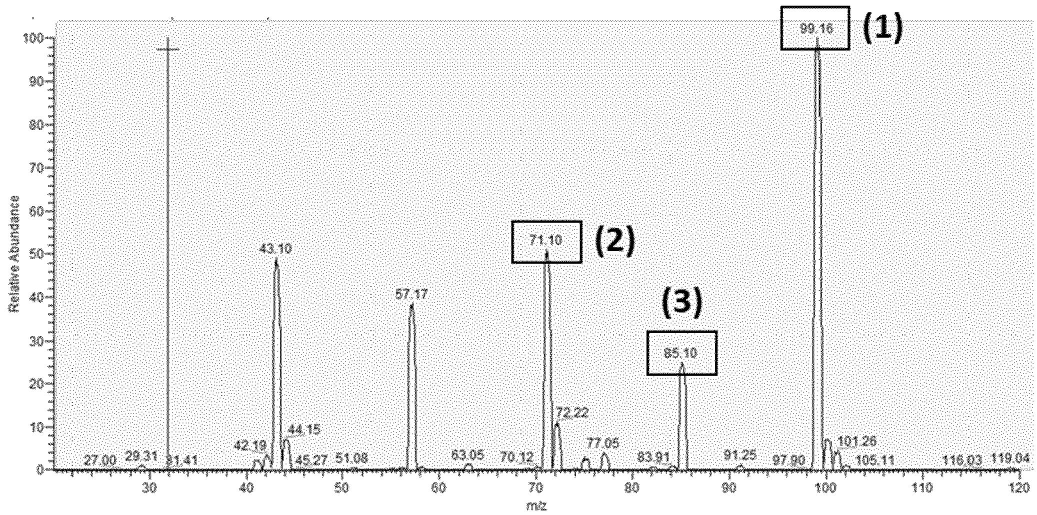

Europäisches Patentamt
European Patent Office
Office européen des brevets

# EUROPEAN SEARCH REPORT

Application Number
EP 21 19 0496

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | WO 2021/160696 A1 (IMMUNDIAGNOSTIK AG [DE]) 19 August 2021 (2021-08-19) * par. [011], [013], [017]-[021], [024], [026], [049],[051], [053]-[056]; the claims * | 1-15 | INV. G01N33/68 |
| A,D | WO 2019/158718 A1 (FROST DIAGNOSTIKA GMBH [DE]) 22 August 2019 (2019-08-22) * claims 1-12 * | 1-15 | |
| A | EP 2 020 446 A1 (VINCENT DENIS [FR]) 4 February 2009 (2009-02-04) * par. [0009]-[0019], [0066]; the claims * | 1-15 | |
| X | PAYNE N A ET AL: "Analysis of histamine and N^@t-methylhistamine in plasma by gas chromatography-negative ion-chemical ionization mass spectrometry", ANALYTICAL BIOCHEMISTRY, ACADEMIC PRESS, AMSTERDAM, NL, vol. 178, no. 2, 1 May 1989 (1989-05-01), pages 414-420, XP024825914, ISSN: 0003-2697, DOI: 10.1016/0003-2697(89)90663-5 [retrieved on 1989-05-01] * abstract; page 415, right-column, 2nd paragraph * | 12,13 | TECHNICAL FIELDS SEARCHED (IPC): G01N |

1 The present search report has been drawn up for all claims

| Place of search | Date of completion of the search | Examiner |
|---|---|---|
| Munich | 27 January 2022 | Lindberg, Pia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 19 0496

This annex lists the patent family members relating to the patent documents cited in the above-mentioned European search report. The members are as contained in the European Patent Office EDP file on
The European Patent Office is in no way liable for these particulars which are merely given for the purpose of information.

27-01-2022

| Patent document cited in search report | Publication date | Patent family member(s) | Publication date |
|---|---|---|---|
| WO 2021160696 A1 | 19-08-2021 | NONE | |
| WO 2019158718 A1 | 22-08-2019 | EP 3752838 A1 | 23-12-2020 |
| | | WO 2019158718 A1 | 22-08-2019 |
| EP 2020446 A1 | 04-02-2009 | AT 495271 T | 15-01-2011 |
| | | CA 2695662 A1 | 05-02-2009 |
| | | EP 2020446 A1 | 04-02-2009 |
| | | EP 2183389 A2 | 12-05-2010 |
| | | US 2010297631 A1 | 25-11-2010 |
| | | WO 2009016231 A2 | 05-02-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5284749 A, Cowley
- US 4818683 A, Morel
- EP 1948819 A
- WO 2019158718 A
- EP 1948819 B1
- US 8003343 B


### Non-patent literature cited in the description


- **CATALDI et al.** Histamine receptors and antihistamines: from discovery to clinical applications. *Chem Immunol Allergy,* 2014, vol. 100, 214-26
- **SATTLER J et al.** Food induced histaminosis as an epidemiological problem: plasma histamine elevation and hemodynamic alterations after oral histamine administration and blockade of diamine oxidase (DAO). *Agents and Actions,* 1988, vol. 23, 361-65
- **WANTKE F et al.** The red wine maximization test: drinking histamine rich wine induces a transient increase of plasma diamine oxidase activity in healthy volunteers. *Inflamm Res,* 1999, vol. 48, 169-70
- **WANTKE F et al.** The red wine provocation test: intolerance to histamine as a model for food intolerance. *Allergy Proc,* 1994, vol. 15, 27-32
- **WANTKE F et al.** Daily variations of serum diamine oxidase and the influence of H1 and H2 blockers: a critical approach to routine diamine oxidase assessment. *Inflamm Res,* 1998, vol. 47, 396-400
- **JARISCH R et al.** Role of food allergy and food intolerance in recurrent urticaria. *Curr Probl Dermatol,* 1999, vol. 28, 64-73
- **WANTKE F et al.** Histamine free diet: treatment of choice for histamine induced food intolerance and supporting treatment for chronical headaches. *Clin Exp Allergy,* 1993, vol. 23, 982-85
- **GÖTZ M et al.** Histamin-Intoleranz und Diaminooxidasemangel. *Allergologie,* 1996, vol. 9, 426-30
- **JARISCH R et al.** Histamin-Intoleranz. Histamin und Seekrankheit. Georg Thieme Verl, 2004
- **MCGRATH AP et al.** Structure and Inhibition of Human Diamine Oxidase. *Biochemistry,* 2009, vol. 48, 9810-22
- **ELMORE BO et al.** Human kidney diamine oxidase: heterologous expression, purification, and characterization. *J Biol Inorg Chem,* 2002, vol. 7, 565-679
- **MIZUGUCHI et al.** Purification and characterization of diamine oxidase (histaminase) from rat small intestine. *J. Biochem,* 1994, vol. 116, 631-5
- **KITANAKA et al.** Expression of diamine oxidase (histaminase) in guinea-pig tissues. *Eur J Pharmacol,* 2002, vol. 437, 179-85
- **KHANDELWAL et al.** Presence and measurement of methylimidazole acetic acids in brain and body fluids. *J Biol Chem,* 10 November 1982, vol. 257 (21), 12815-9
- **TSURUTA et al.** Simultaneous determination of imidazole acetic acid and N tau- and N pi-methylimidazole acetic acids in human urine by high-performance liquid chromatography with fluorescence detection. *J Chromatogr,* 24 April 1987, vol. 416 (1), 63-9
- **PREUSS et al.** Human histamine N-methyl-transferase pharmaco-genetics: common genetic polymorphisms that alter activity. *Molecular Pharmacology,* 01 April 1998, vol. 53 (4), 708-717
- **RUTHERFORD et al.** The histamine N-methyltransferase T105I polymorphism affects active site structure and dynamics. *Biochemistry,* 22 January 2008, vol. 47 (3), 893-901
- **LEE HS et al.** Involvement of human histamine N-methyl-transferase gene poly-morphisms in susceptibility to atopic dermatitis in korean children. *Allergy Asthma Immunol Res,* January 2012, vol. 4 (1), 31-6
- **NELIS et al.** Development of a HILIC-MS/MS method for the quantification of histamine and its main metabolites in human urine samples. *Talanta,* 01 December 2020, vol. 220, 121328
- **ELLMAN GL.** *Arch Biochem Biophys,* 1958, vol. 74, 443-450
- **RIDDLES PW.** *Anal Biochem,* 1979, vol. 94, 75-81
- **MUCKERHEIDE A et al.** *J. Immunol,* 1987, vol. 138, 833
- **APPLE RJ et al.** *J Immunol,* 1987, vol. 140, 290
- **DOMEN PJ.** *J Immunol,* 1987, vol. 139, 195
- **MOREL ; DELAAGE.** Immuno analysis of histamine through a novel chemical derivatization. *The Journal of Allergy and Clinical Immunology,* 1988, vol. 82, 646-654

- **MOREL et al.** Recognition of imidazole and histamine derivatives by monoclonal antibodies. *Immunol,* 1990, vol. 27, 995-1000
- **HELD, P. et al.** Analysis of Histamine in Wine Samples Using the Microplate Format. *BioTek Instruments,* 28 August 2006
- **MAYER I et al.** Optimierter Radioextraktionsassay zur quantitativen Bestimmung der Aktivität von Diaminooxidase (DAO) in humanem Serum und Plasma. *Allergologie,* 2005, vol. 28, 1-8
- *Appl Biochem Biotechnol,* November 2007, vol. 143 (2), 164-75
- **HIBI T et al.** Enzymatic assay of histamine by amperometric detection of H2O2 with a peroxidase-based sensor. *Biosci Biotechnol Biochem,* 2000, vol. 64 (9), 1963-1966
- **KEHOE CA et al.** Plasma diamine oxidase activity is greater in copper-adequate than copper-marginal or copper-deficient rats. *J. Nutr,* 2000, vol. 130, 30-33
- **KÜFNER MA et al.** Total histamine degradation capacity (THDC) as an important biological marker of histamine metabolism in human colonic mucosa. *Inflamm Res,* 2002, (1), 87-81
- **NILSSON B-O et al.** Inhibition of diamine oxidase promotes uptake of putrescine from rat small intestine. *Inflamm Res,* 1996, vol. 45, 513-518
- **NOVOTNY et al.** Diamine oxidase is the amiloride-binding protein and is inhibited by biological marker of histamine metabolism in human colonic mucosa. *Inflamm Res,* 2002, (1), 87-81
- **NOVOTNY et al.** Diamine oxidase is the amiloride-binding protein and is inhibited by products. *Eur J Biochem,* 2004, vol. 271, 146-152
- **ZIMATKIN SM et al.** Alcohol-histamine interactions. *Alcohol Alcohol,* 1999, vol. 34 (2), 151-7